# EUROPEAN PATENT APPLICATION

(11) **EP 4 796 640 A1**
(43) Date of publication of application: **26.08.2026**
(21) Application number: 24890881.6
(22) Date of filing: 13.11.2024
(51) Int. Cl.: C12N 15/82

(54) **PLANT ENRICHED WITH AMINO ACIDS, PROTEINS AND STARCH**

(30) Priority: 14.11.2023 ES 202330933
(71) Applicant: Universitat de Valéncia, 46010 Valencia (ES)
(72) Inventor: ROS PALAU, Roque Luis, 46006 Valencia, Valencia (ES); MUÑOZ BERTOMEU, Jesús, 46100 Burjassot, Valencia (ES); CASATEJADA ANCHEL, Rubén, 46900 Torrent, Valencia (ES); TORRES MONCHO, Alejandro, 03700 Denia, Alicante (ES); ROSA TÉLLEZ, Sara Virginia, 29680 Estepona, Málaga (ES); ALCÁNTARA ENGUÍDANOS, Andrea, 46132 Almàssera, Valencia (ES); RENAU MORATA, Begoña, 46001 Valencia, Valencia (ES); DOGHRI, Maroua, 46100 Burjassot, Valencia (ES)
(74) Representative: Manuel Illescas y Asociados, S.L.U.
(86) International application number: PCT/ES2024/070702
(87) International publication number: WO 2025/104359

(57) **Abstract**

The present invention relates to a plant or plant cell that is genetically modified to overexpress a gene that codes for the D-3-phosphoglycerate dehydrogenase (PGDH1) protein, wherein PGDH1 comprises amino acids conserved between species and wherein said plant does not belong to the *Arabidopsis thaliana* species. The invention further relates to a method that comprises transforming wild type plants or plant cells with a recombinant expression vector. The invention also relates to uses of the plant or plant cell for producing proteins, amino acids and/or starch.

## Description

### FIELD OF THE INVENTION

The present invention relates generally to the field of genetically modified plants. In particular, the present invention relates to genetically modified plants to overexpress a gene encoding the D-3-phosphoglycerate dehydrogenase (PGDH1) protein. These plants have a higher content of amino acids, proteins and starch compared to untransformed plants.

### BACKGROUND OF THE INVENTION

Food of plant origin are considered healthy and can help prevent disease, but they lack sufficient protein content to maintain a balanced diet in humans. Crop plants, and especially cereals, are low in protein. In corn grain, for example, the protein content is only 10%. This low protein content is aggravated by climate change since it has been observed that an increase in CO₂ concentration increases the carbohydrate content to the detriment of the protein content of vegetables (Mizokami et al. (2019); Myers et al. (2019)). This lack of protein content also appears in livestock species, where the foods of plant origin supplied are enriched through the addition of amino acids, which increases livestock nutrition expenses for farmers.

Furthermore, foods of plant origin have advantages over foods of animal origin. The excessive consumption of products of animal origin, especially red meats and processed meats, a traditional source of protein incorporation into the human diet, can have negative effects on health, by increasing the risk of diseases such as diabetes, heart disease, strokes and cancer.

Moreover, the production of proteins of animal origin is much more expensive than the production of proteins of plant origin and has a much higher environmental cost. It is also one of the main causes of soil and water resource degradation.

The increase in the nutritional value of plants by increasing their protein or carbohydrate content is of great interest, specifically for the agri-food industry. There is intense research work in the search for alternative sources of protein. High protein or carbohydrate plant products can be the basis for developing new foods with added nutritional value that can be enriched in one or another metabolite according to requirements.

The development of plants with greater efficiency in protein biosynthesis is highly necessary since it can increase protein production at low economic and environmental cost from cereals and other low protein plant crops, in addition to contributing to the improvement of health by reducing the need for excessive consumption of animal products. It can also help to improve the nutrition of vulnerable populations with little access to products of animal origin and with malnutrition problems (groups whose diet is solely cereal-based).

Humans and other monogastric animals cannot synthesise the so-called essential amino acids, which must be supplied by the diet. Among them the most limiting in plants, and especially in cereals with respect to the nutritional needs of humans and animals are lysine, methionine, threonine and tryptophan, which has a negative impact on the nutritional quality of the plants. The development of plants with higher content in some of these amino acids, such as lysine, methionine and threonine is greatly necessary.

Genetically modified plants that have been developed to date have not achieved sufficiently satisfactory results. In some instances, the genetically modified plants exhibited undesirable alterations in their development.

### DESCRIPTION OF THE INVENTION

For the purposes of the present invention, "complementary DNA (cDNA)" refers to a doublestranded DNA molecule, wherein one of its strands constitutes a sequence fully complementary to the messenger RNA wherefrom it has been synthesised. It is used in plant transformation, because, given the nature of its synthesis, it lacks introns.

For the purposes of the present invention, "plant with C₃ metabolism" refers to a plant whose metabolic pathway for carbon fixation during photosynthesis is a 3-carbon pathway. In the 3-carbon pathway, CO₂ is directly fixed in the Calvin cycle, with no previous fixations occurring. The first stable product wherein the carbon in the Calvin cycle is fixed is 3-phosphoglycerate, a 3-carbon compound, which explains the name of C₃ metabolism.

For the purposes of the present invention, "plant with C₄ metabolism" refers to a plant whose metabolic pathway for carbon fixation during photosynthesis is a 4-carbon pathway or Hatch-Slack pathway. In the 4-carbon pathway, prior to the carboxylation of ribulose-1,5-bisphosphate catalysed by the Rubisco enzyme, which produces 3-phosphoglycerate of three carbon atoms, a first carboxylation of phosphoenolpyruvic acid (PEP) occurs, resulting in a dicarboxylic acid of four carbon atoms (malic acid or aspartic acid) as the primary stable product. This type of plant avoids the loss of CO₂ in photorespiration insofar as possible.

The existing problem in the state of the art is to provide a genetically modified plant with improved efficiency in amino acid, protein and starch biosynthesis.

Additionally, in particular, the problem existing in the state of the art consists of providing a genetically modified plant with an improved efficiency in the biosynthesis of amino acids, proteins and starch under conditions of elevated environmental CO₂.

The present invention solves both technical problems by means of a genetically modified plant to overexpress a gene encoding a D-3-phosphoglycerate dehydrogenase protein (PGDH1; EC 1.1.1.95), a protein of the phosphorylation pathway of serine biosynthesis.

Three enzymes that catalyse the conversion of D-3-phosphoglycerate to serine are involved in the phosphorylation pathway of serine biosynthesis: D-3-phosphoglycerate dehydrogenase (PGDH1; EC 1.1.1.95), phosphoserine aminotransferase (PSAT; EC 2.6.1.52), and phosphoserine phosphatase (PSP; EC 3.1.3.3). In *Arabidopsis thaliana* there are three isoforms of the PGDH protein: PGDH1 isoform, encoded by the *PGDH1* gene *(*also referred to as *EDA9*), PGDH2 isoform, encoded by the *PGDH2* gene ( also referred to as *PGDH*), and PGDH3 isoform, encoded by the *PGDH3* gene *(*also referred to as *3-PGDH*). The three isoforms have a percentage of identity at the amino acid level of more than 74%.

The present invention provides a genetically modified plant, not constituting a Plant Variety, or a genetically modified plant cell, for overexpressing a gene encoding a D-3-phosphoglycerate dehydrogenase (PGDH1) protein, wherein the amino acid sequence of said PGDH1 protein comprises the following amino acids:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348;
wherein said positions refer to the position in the amino acid sequence of the *Arabidopsis thaliana* PGDH1 protein identified by the sequence SEQ ID NO: 1; and wherein said plant does not belong to the *Arabidopsis thaliana species.*

The amino acids in the above paragraph include individual conserved amino acids of the enzyme. Said conserved individual amino acids can be grouped, in turn, into the following conserved domains of the enzyme, essential for its activity:
- leucine, at position 65;
- leucine at position 78;
- domain constituted by arginine and serine at positions 108 and 109 respectively; followed by threonine at position 111;
- alanine and alanine at positions 142 and 143, respectively;
- alanine and glutamic acid at positions 162 and 163, respectively;
- domain constituted by arginine and glycine at positions 291 and 292, respectively; followed by leucine at position 300;
- domain constituted by aspartic acid, valine and phenylalanine at positions 315, 316 and 317, respectively; followed by glutamic acid and proline at positions 320 and 321, respectively; and
- domain constituted by proline and histidine at positions 338 and 339, respectively; followed by glycine at position 341; serine and threonine at positions 343 and 344, respectively; and, finally, glutamic acid, alanine and glutamine at positions 346, 347 and 348, respectively;

After performing an analysis of the sequences of plants (*Arabidopsis thaliana*, tomato and corn), bacteria (*Escherichia coli* and *Mycobacterium tuberculosis*) and mammals (*Homo sapiens* and *Rattus norvegicus*), the inventors have determined that the amino acids and domains of the previous paragraph are conserved in all the species studied including bacteria and mammals and are responsible for the D-3-phosphoglycerate dehydrogenase function, synthesising serine, being conserved in all species including bacteria and mammals. Since these domains are conserved in plants, bacteria and mammals, they have a high biological significance and a high functional importance.

The genetically modified plant or plant cell of the invention overexpresses a gene encoding a D-3-phosphoglycerate dehydrogenase (PGDH1) protein with the amino acids and domains conserved in all species studied including bacteria and mammals and is, therefore, an active protein for D-3-phosphoglycerate dehydrogenase function, synthesising serine.

In a preferred embodiment of the invention, the amino acid sequence of said PGDH1 protein further comprises the following amino acids:
- glycine at position 209;
- glycine at position 212;
- aspartate at position 256; and
- histidine at position 261;
wherein said positions refer to the position in the amino acid sequence of the *Arabidopsis thaliana* PGDH1 protein identified by the sequence SEQ ID NO: 1.

Thus, in a preferred embodiment, the amino acid sequence of said PGDH1 protein comprises the following amino acids:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- glycine at position 209;
- glycine at position 212;
- aspartate at position 256;
- histidine at position 261;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348;
wherein said positions refer to the position in the amino acid sequence of the *Arabidopsis thaliana* PGDH1 protein identified by the sequence SEQ ID NO: 1; and wherein said plant does not belong to the *Arabidopsis thaliana* species*.*

The amino acids of the previous paragraph also include conserved individual amino acids of the enzyme. Such conserved individual amino acids can be grouped, in turn, into the following conserved domains of the enzyme:
- glycine and glycine at positions 209 and 212, respectively;
- aspartate and histidine at positions 256 and 261, respectively; and
- arginine and glycine at positions 291 and 292, respectively; followed by leucine at position 300.

In a preferred embodiment, the present invention provides a plant, not constituting a Plant Variety, or a genetically modified plant cell, to overexpress a gene encoding the D-3-phosphoglycerate dehydrogenase (PGDH1) protein, wherein the amino acid sequence of said PGDH1 protein has at least 65% identity to the sequence of the *Arabidopsis thaliana* PGDH1 protein (SEQ ID NO: 1) wherein said plant does not belong to the *Arabidopsis thaliana* species*.*

Overexpression of the gene encoding the D-3-phosphoglycerate dehydrogenase (PGDH1) protein leads to a significant increase in the amino acid, protein and starch content in the plants thus modified. The PGDH1 protein is a protein of the phosphorylation pathway of serine biosynthesis, which is a general pathway in all plants. Thus, the present invention provides a solution for increasing the content of amino acids, proteins and starch in numerous species of agronomic interest, including cereals. The present invention is especially relevant in the new climate change conditions where elevated levels of ambient CO₂ are expected. In fact, a very remarkable advantage of the present invention is that it manages to increase the production of proteins and starch under plant growth conditions wherein there are elevated CO₂ concentrations. The increase in protein production obtained is an unexpected result since an increase in CO₂ concentration increases the starch content in the plants while reducing their protein content. Under such elevated CO₂ conditions, the present invention also manages to significantly increase the majority of the essential amino acids (leucine, isoleucine, phenylalanine, valine, threonine, lysine and methionine) in plants. This increase in the production of essential amino acids is an unexpected result in view of the fact that, as previously indicated, an increase in CO₂ concentration reduces the protein content of plants (Myers et al. (2019)).

The results obtained by the inventors demonstrate that the present invention directs the plant metabolism towards an increased content of some essential amino acids and, in turn, increases the generalised production of proteins and carbohydrates, and not only to a greater production of a specific protein or amino acid. In principle, therefore, the present invention comprises any plant since the phosphorylation pathway of serine biosynthesis, involving PGDH1, is a general pathway to all of them.

Another advantage of the present invention is that, in contrast to other strategies of the state of the art, there is no reduction in the growth of the plant of the invention.

Hence, it follows that the present invention avoids the effect of climate change on the nutritional quality of crops and increases the production of proteins with low economic and environmental cost. On the other hand, the present invention allows reducing the consumption of proteins of animal origin in humans and in livestock species.

**Table 1** shows information on the sequences SEQ ID NO: 1-9.

**Table 1**

| **Sequence** | **Sequence number** |
|---|---|
| Amino acid sequence of the *Arabidopsis thaliana* PGDH1 protein | SEQ ID NO: 1 |
| Amino acid sequence corresponding to amino acids 61 to 348 of the *Arabidopsis thaliana* PGDH1 protein | SEQ ID NO: 2 |
| Amino acid sequence corresponding to amino acids 127 to 140 of the *Arabidopsis thaliana* PGDH1 protein | SEQ ID NO: 3 |
| Amino acid sequence corresponding to amino acids 147 to 158 of the *Arabidopsis thaliana* PGDH1 protein | SEQ ID NO: 4 |
| Amino acid sequence corresponding to amino acids 192 to 204 of the *Arabidopsis thaliana* PGDH1 protein | SEQ ID NO: 5 |
| Amino acid sequence corresponding to amino acids 423 to 433 of the *Arabidopsis thaliana* PGDH1 protein | SEQ ID NO: 6 |
| Amino acid sequence corresponding to amino acids 532 to 544 of the *Arabidopsis thaliana* PGDH1 protein | SEQ ID NO: 7 |
| Nucleotide sequence of complementary DNA of the *Arabidopsis thaliana* PGDH1 gene | SEQ ID NO: 8 |
| Nucleotide sequence of the *Arabidopsis thaliana PGDH1* gene | SEQ ID NO: 9 |

The nucleotide sequence of the complementary DNA (cDNA) of the *Arabidopsis thaliana PGDH1* gene is the sequence SEQ ID NO: 8. The reference number of the nucleotide sequence of the *Arabidopsis thaliana PGDH1* gene in the database "The Arabidopsis Information Resource", which is the reference database for *Arabidopsis thaliana*, is AT4G34200. The sequence SEQ ID NO: 8 corresponds to said sequence with reference number AT4G34200 in "The Arabidopsis Information Resource".

The nucleotide sequence of the *Arabidopsis thaliana PGDH1* gene encodes the PGDH1 protein, the amino acid sequence of which is SEQ ID NO: 1. The reference number of the amino acid sequence of the *Arabidopsis thaliana* PGDH1 protein in the "National Center for Biotechnology Information" is NP_195146.1. The sequence SEQ ID NO: 1 corresponds to said sequence with reference number NP_195146.1 in the "National Center for Biotechnology Information".

The sequence SEQ ID NO: 2 corresponds to amino acids 61 to 348 of the *Arabidopsis thaliana* PGDH1 protein.

In a preferred embodiment of the plant or plant cell of the invention, the amino acid sequence of the PGDH1 protein has an identity to the sequence of the *Arabidopsis thaliana* PGDH1 protein (SEQ ID NO: 1) of at least 66%, of at least 67%, of at least 68%, of at least 69%, of at least 70%, of at least 71%, of at least 72%, of at least 73%, of at least 74%, of at least 75%, of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

In another preferred embodiment of the plant or plant cell of the invention, said PGDH1 protein comprises a sequence having an identity of at least 75% to the sequence SEQ ID NO: 2.

Preferably, said PGDH1 protein comprises a sequence having an identity to the sequence SEQ ID NO: 2 of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

The inventors have conducted an analysis of the sequences of the PGDH1 protein in the plants *Arabidopsis thaliana*, tomato (*Solanum lycopersicum*), corn (*Zea mays*) and other species of agronomic interest. The results are shown in **Table 2**. In all the cases studied from different plant species, a sequence identity higher than 65% has been found.

**Table 2**

| **Scientific name** | **Percentage of identity** | **Length** | **Reference number (NCBI)** |
|---|---|---|---|
| *Brassica rapa* | 92.87% | 596 | XP_009115555.2 |
| *Brassica napus* | 93.17% | 602 | XP_013675915.2 |
| *Hordeum vulgare* subsp. vulgare | 83.43% | 617 | XP_044968223.1 |
| *Oryza sativa* Japonica Group | 82.50% | 613 | XP_015635449.1 |
| *Hordeum vulgare* subsp*.* vulgare | 77.31% | 672 | XP_044957600.1 |
| *Solanum lycopersicum* | 79.63% | 595 | XP_004248761.1 |
| *Triticum aestivum* | 82.87% | 617 | XP_044459395.1 |
| *Zea mays* | 82.14% | 618 | PWZ37369.1 |

The inventors have also performed an analysis of the sequences of amino acids 61 to 348 of the PGDH1 protein in *Arabidopsis thaliana* plants, tomato (*Solanum lycopersicum*), corn (*Zea mays*) and other species of agronomic interest. The results are shown in **Table 3**. In all the cases studied from different plant species, a sequence identity higher than 75% has been found.

**Table 3**

| **Scientific name** | **Percentage of identity** | **Length** | **Reference number (NCBI)** |
|---|---|---|---|
| *Brassica napus* | 79.86% | 621 | XP_013687658.1 |
| *Brassica oleracea* | 94.10% | 602 | VDD00124.1 |
| *Brassica oleracea* var. oleracea | 79.86% | 621 | XP_013604677.1 |
| *Brassica rapa* | 96.18% | 596 | XP_009115555.2 |
| *Brassica rapa* | 79.51% | 621 | XP_009110390.1 |
| *Hordeum vulgare* | 85.42% | 404 | KAE8798432.1 |
| *Oryza sativa* Japonica Group | 84.03% | 613 | XP_015635449.1 |
| *Solanum lycopersicum* | 87.50% | 600 | XP_004236276.1 |
| *Solanum lycopersicum* | 87.50% | 595 | XP_004248761.1 |
| *Triticum aestivum* | 85.07% | 618 | XP_044326513.1 |
| *Triticum aestivum* | 84.72% | 617 | XP_044459395.1 |
| *Zea mays* | 83.33% | 618 | PWZ37369.1 |
| *Zea mays* | 82.64% | 612 | ACG25573.1 |

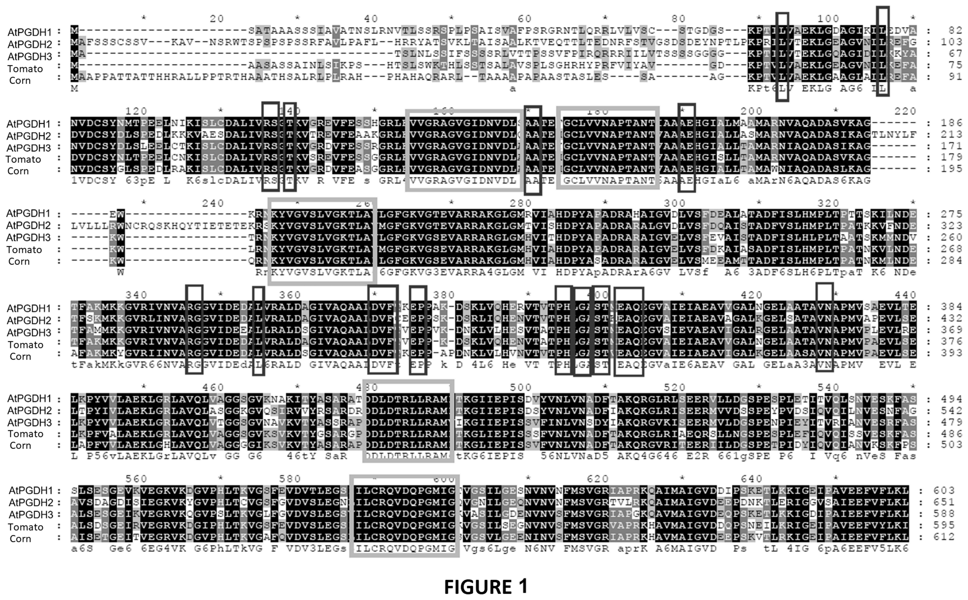

Additionally, the inventors have performed a sequence alignment of the proteins PGDH1, PGDH2 and PGDH3 from *Arabidopsis thaliana*, PGDH from tomato and PGDH from corn, shown in **Figure 1**.

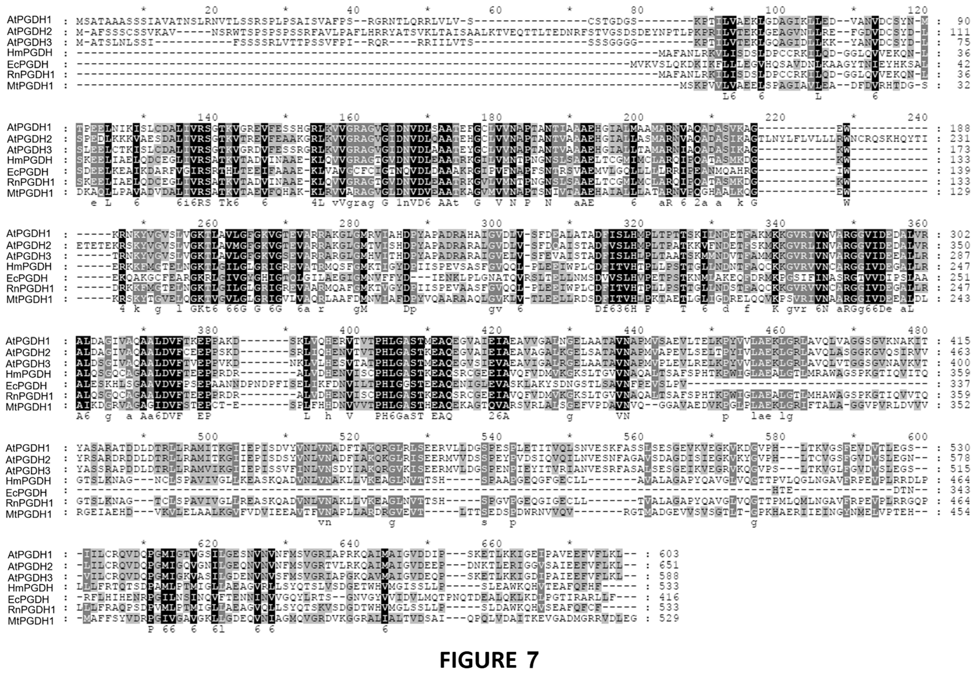

The analysis made it possible to determine that several amino acids of the PGDH1 protein are conserved in plants (*Arabidopsis thaliana*, tomato and corn), in bacteria (*Escherichia coli* and *Mycobacterium tuberculosis*) and in mammals (*Homo sapiens* and *Rattus norvegicus*). The sequence alignment of the *Arabidopsis thaliana* PGDH1, PGDH2 and PGDH3, *Homo sapiens* PGDH, *Escherichia coli* PGDH, rat PGDH2 (*Rattus norvegicus*) and *Mycobacterium tuberculosis* PGDH1 proteins are shown in **Figure 7**. These amino acids are the following:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348.

The positions indicated in the previous paragraph refer to the position in the amino acid sequence of the *Arabidopsis thaliana* PGDH1 protein.

Thus, in another preferred embodiment of the plant or plant cell of the invention, in said PGDH1 protein at least one of the amino acids is:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348.

In another preferred embodiment of the plant or plant cell of the invention, the amino acid sequence of said PGDH1 protein comprises a sequence selected from the group consisting of: SEQ ID NO: 3, SEQ ID NO: 4, SEQ ID NO: 5, SEQ ID NO: 6 and SEQ ID NO: 7, which correspond to PGDH1 protein sequences conserved only in plants (they are conserved in *Arabidopsis thaliana*, tomato and corn), but not conserved either in bacteria (*Escherichia coli* and *Mycobacterium tuberculosis*), or in mammals (*Homo sapiens* and *Rattus norvegicus*).

In another preferred embodiment of the plant or plant cell of the invention, the amino acid sequence of said PGDH1 protein comprises a sequence selected from the group consisting of: SEQ ID NO: 16, SEQ ID NO: 17, SEQ ID NO: 18 and SEQ ID NO: 19. The sequences SEQ ID NO: 16, SEQ ID NO: 17, SEQ ID NO: 18 and SEQ ID NO: 19 correspond to:
- Amino acids 203 to 230 of the *Arabidopsis thaliana* PGDH1 protein (SEQ ID NO: 16)
- Amino acids 251 to 273 of the *Arabidopsis thaliana* PGDH1 protein (SEQ ID NO: 17)
- Amino acids 280 to 296 of *Arabidopsis thaliana* PGDH1 protein (SEQ ID NO: 18)
- Amino acids 530 to 603 of the *Arabidopsis thaliana* PGDH1 protein (SEQ ID NO: 19)

In a more preferred embodiment of the plant or plant cell of the invention, the amino acid sequence of said PGDH1 protein is the sequence SEQ ID NO: 1.

In a preferred embodiment, the plant or plant cell of the invention has a higher content of amino acids and/or proteins and/or starch than a non-transformed plant or plant cell.

In a more preferred embodiment, the plant or plant cell of the invention has an at least 5% higher content of amino acids and/or at least 5% higher content of proteins and/or at least 5% higher content of starch with respect to an untransformed plant or plant cell.

In an even more preferred embodiment, the plant of the invention or the plant cell has an at least 5% higher content of amino acids and/or at least 5% higher content of proteins and/or at least 5% higher content of starch with respect to an untransformed plant or plant cell, under culture conditions wherein the CO₂ concentration is at least 800 ppm. Currently, CO₂ values in the atmosphere are significantly lower than 800 ppm. However, in several future scenarios, CO₂ values in the atmosphere could exceed 800 ppm.

In another preferred embodiment, the plant or plant cell of the invention is from a species of agronomic interest.

In a more preferred embodiment, the plant or plant cell of the invention is from a species with C₃ metabolism. Preferably, the species with C₃ metabolism is selected from tomato or broccoli.

In a more preferred embodiment, the plant or plant cell is from a species with C₄ metabolism. Preferably, the species with C₄ metabolism is corn.

In another preferred embodiment of the plant or plant cell of the invention, the nucleotide sequence of the gene encoding the PGDH1 protein has an identity of at least 65% to the sequence SEQ ID NO: 8 or respect to the sequence SEQ ID NO: 9.

Preferably, the nucleotide sequence of the gene encoding the PGDH1 protein has an identity to the sequence SEQ ID NO: 8 or to the sequence SEQ ID NO: 9 of at least 66%, of at least 67%, of at least 68%, of at least 69%, of at least 70%, of at least 71%, of at least 72%, of at least 73%, of at least 74%, of at least 75%, of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

In a more preferred embodiment of the plant or plant cell of the invention, the nucleotide sequence of the gene encoding the PGDH1 protein is the sequence SEQ ID NO: 8 or the sequence SEQ ID NO: 9.

In another preferred embodiment, the gene encoding the PGDH1 protein is operably linked to a constitutive promoter. Preferably, the promoter is selected from the constitutive promoter of the gene encoding corn ubiquitin and the constitutive promoter of cauliflower mosaic virus 35S.

In another preferred embodiment, the plant of the invention does not undergo alteration in its growth as a plant, in relation to the growth, under identical cultivation conditions, of an untransformed plant, of the same species.

In another preferred embodiment, the plant of the invention does not exhibit an increase in root size relative to the size of the roots of an untransformed plant.

In another preferred embodiment, the plant of the invention does not exhibit an increase in the size of the aerial part relative to the size of the aerial part of an untransformed plant.

The present invention also provides a recombinant expression construct comprising a gene encoding the D-3-phosphoglycerate dehydrogenase (PGDH1) protein, wherein the amino acid sequence of said PGDH1 protein has at least 65% identity with the sequence SEQ ID NO: 1 and wherein the gene encoding the PGDH1 protein is operably linked to a constitutive promoter.

In a preferred embodiment of the recombinant expression construct of the invention, the constitutive promoter is selected from among the constitutive promoter of the gene encoding corn ubiquitin and the constitutive promoter of the cauliflower mosaic virus *35S*.

In another preferred embodiment of the recombinant expression construct of the invention, the nucleotide sequence of the gene encoding the PGDH1 protein has an identity of at least 65% to the sequence SEQ ID NO: 8 or to the sequence SEQ ID NO: 9.

Preferably, the nucleotide sequence of the gene encoding the PGDH1 protein has an identity to the sequence SEQ ID NO: 8 or to the sequence SEQ ID NO: 9 of at least 66%, of at least 67%, of at least 68%, of at least 69%, of at least 70%, of at least 71%, of at least 72%, of at least 73%, of at least 74%, of at least 75%, of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

In a more preferred embodiment of the recombinant expression construct of the invention, the nucleotide sequence of the gene encoding the PGDH1 protein is the sequence SEQ ID NO: 8 or the sequence SEQ ID NO: 9.

In another preferred embodiment of the method of the invention, the amino acid sequence of said PGDH1 protein comprises a sequence selected from the group consisting of: SEQ ID NO: 16, SEQ ID NO: 17, SEQ ID NO: 18 and SEQ ID NO: 19.

The present invention also provides a recombinant expression vector comprising the recombinant expression construct of the invention.

In a preferred embodiment of the recombinant expression vector of the invention, the nucleotide sequence of the gene encoding the PGDH1 protein has an identity of at least 65% to the sequence SEQ ID NO: 8 or to the sequence SEQ ID NO: 9.

Preferably, the nucleotide sequence of the gene encoding the PGDH1 protein has an identity to the sequence SEQ ID NO: 8 or to the sequence SEQ ID NO: 9 of at least 66%, of at least 67%, of at least 68%, of at least 69%, of at least 70%, of at least 71%, of at least 72%, of at least 73%, of at least 74%, of at least 75%, of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

In a more preferred embodiment of the recombinant expression vector of the invention, the nucleotide sequence of the gene encoding the PGDH1 protein is the sequence SEQ ID NO: 8 or the sequence SEQ ID NO: 9.

The present invention further provides a non-essentially biological method for obtaining a genetically modified plant, or a genetically modified plant cell, for overexpressing a gene encoding a D-3-phosphoglycerate dehydrogenase (PGDH1) protein, that comprises transforming the wild type plants or the corresponding plant cells with an expression vector comprising a gene encoding the D-3-phosphoglycerate dehydrogenase (PGDH1) protein, wherein the amino acid sequence of said PGDH1 protein comprises the following amino acids:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348;
wherein said positions refer to the position in the amino acid sequence of the *Arabidopsis thaliana* PGDH1 protein identified by the sequence SEQ ID NO: 1; and wherein said plant or plant cell does not belong to the *Arabidopsis thaliana* species.

In a preferred embodiment of the method of the invention, the amino acid sequence of said PGDH1 protein further comprises the following amino acids:
- glycine at position 209;
- glycine at position 212;
- aspartate at position 256; and
- histidine at position 261;
wherein said positions refer to the position in the amino acid sequence of the *Arabidopsis thaliana* PGDH1 protein identified by the sequence SEQ ID NO: 1.

In a preferred embodiment, the present invention provides a non-essentially biological method for obtaining a genetically modified plant or a genetically modified plant cell that comprises transforming the wild type plants or the corresponding plant cells with an expression vector comprising a gene encoding the D-3-phosphoglycerate dehydrogenase (PGDH1) protein, wherein the amino acid sequence of said PGDH1 protein has an identity of at least 65% to the sequence SEQ ID NO: 1 and wherein said plant or plant cell does not belong to the *Arabidopsis thaliana* species.

Preferably, the amino acid sequence of said PGDH1 protein has an identity to the sequence SEQ ID NO: 1 of at least 66%, of at least 67%, of at least 68%, of at least 69%, of at least 70%, of at least 71%, of at least 72%, of at least 73%, of at least 74%, of at least 75%, of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

The present invention also provides a non-essentially biological method of obtaining a genetically modified plant or a genetically modified plant cell that comprises performing gene editing of the corresponding wild type plants or plant cells to overexpress the gene encoding the D-3-phosphoglycerate dehydrogenase (PGDH1) protein. Preferably, the gene editing is a CRISPR-cas9-based technology.

In a preferred embodiment of the method of the invention, said PGDH1 protein comprises a sequence which has at least 75% identity to the sequence SEQ ID NO: 2. Preferably, said sequence identity is at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

In another preferred embodiment of the method of the invention, in said PGDH1 protein at least one of the amino acids is:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348.

In another preferred embodiment of the method of the invention, the amino acid sequence of said PGDH1 protein comprises a sequence selected from the group consisting of: SEQ ID NO: 3, SEQ ID NO: 4, SEQ ID NO: 5, SEQ ID NO: 6 and SEQ ID NO: 7.

In another preferred embodiment of the method of the invention, the amino acid sequence of said PGDH1 protein is the sequence SEQ ID NO: 1.

In another preferred embodiment, the plant or plant cell is from a species of agronomic interest.

In another preferred embodiment, the plant or plant cell is from a species with C₃ metabolism. Preferably, the species with C₃ metabolism is selected from tomato or broccoli.

In another preferred embodiment, the plant or plant cell is from a species with C₄ metabolism. Preferably, the species with C₄ metabolism is corn.

In another preferred embodiment of the method of the invention, the nucleotide sequence of the gene encoding the PGDH1 protein has an identity of at least 65% to the sequence SEQ ID NO: 8 or to the sequence SEQ ID NO: 9.

Preferably, the nucleotide sequence of the gene encoding the PGDH1 protein has an identity of at least 65% to the sequence SEQ ID NO: 8 or to the sequence SEQ ID NO: 9 of at least 66%, of at least 67%, of at least 68%, of at least 69%, of at least 70%, of at least 71%, of at least 72%, of at least 73%, of at least 74%, of at least 75%, of at least 76%, of at least 77%, of at least 78%, of at least 79%, of at least 80%, of at least 81%, of at least 82%, of at least 83%, of at least 84%, of at least 85%, of at least 86%, of at least 87%, of at least 88%, of at least 89%, of at least 90%, of at least 91%, of at least 92%, of at least 93%, of at least 94%, of at least 95%, of at least 96%, of at least 97%, of at least 98%, or of at least 99%.

In a more preferred embodiment of the method of the invention, the nucleotide sequence of the gene encoding the PGDH1 protein is the sequence SEQ ID NO: 8 or the sequence SEQ ID NO: 9.

The present invention also provides the use of the plant of the invention, of the plant cell of the invention, of the recombinant expression construct of the invention, or of the recombinant expression vector of the invention, for producing proteins, amino acids and/or starch.

In a preferred embodiment of the use of the invention, the plant cell or plant is grown under conditions wherein the CO₂ concentration is at least 800 ppm.

In relation to the percentages of sequence identity of the present invention, the person skilled in the art can determine whether a sequence has at least a certain percentage of identity to a sequence of the present invention, using sequence alignment computer programs known in the state of the art.

For example, it is understood herein that a query sequence has at least 65% identity to a reference sequence of the invention when the sequence may include up to 35 different residues per 100 residues of the reference sequence, either by deletion, insertion or substitution. This extends, analogously, to the other percentages of identity described herein.

Throughout the description and the claims, the term "comprising", "that comprises" and their variants are meant in a non-limiting sense and therefore should not exclude other technical features. The term "comprises", "comprising" and its variants, throughout the description and claims, specifically includes the term "consists of", "consisting of" and their variants.

As used herein and in the claims, the singular form "the" includes references to plural forms unless the content clearly indicates otherwise.

Unless defined otherwise, all the technical and scientific terms used throughout the description and claims have the same meaning as those customarily understood by a person skilled in the field of the invention.

### BRIEF DESCRIPTION OF THE DRAWINGS

**Figure 1****.** Sequence alignment of *Arabidopsis thaliana* PGDH1, PGDH2, and PGDH3, tomato PGDH, and corn PGDH proteins. The domains conserved in the plant species studied have been highlighted in light grey, and in dark grey the domains conserved even in mammals (in rat and in human; the PGDH protein sequences of these two mammalian species are not shown in the figure).

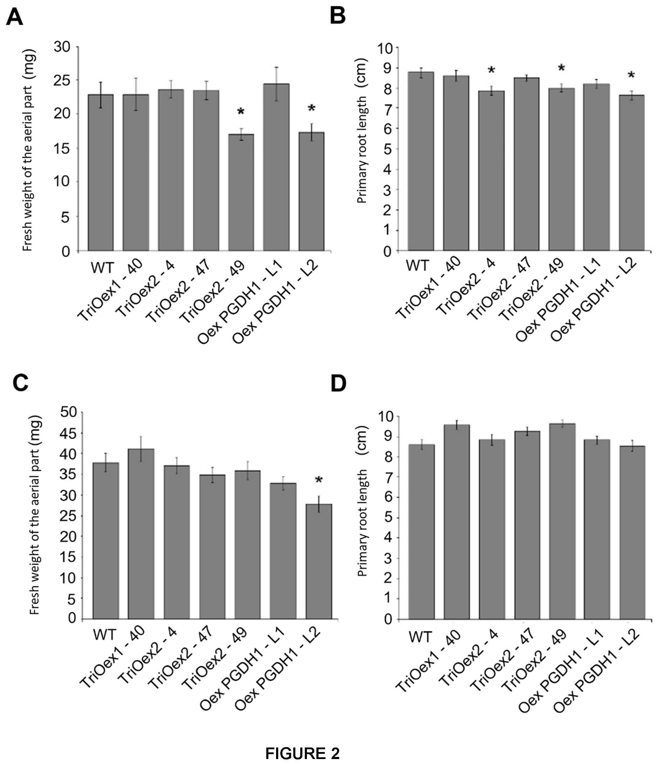

**Figure 2****.** Phenotypic characterisation of triple overexpression (TriOex) and *PGDH1* gene overexpression (Oex PGDH1) lines under ambient CO₂ (400 ppm) (A and B) and elevated CO₂ (2500 ppm) (C and D) conditions of 14-day-old *Arabidopsis thaliana* seedlings grown *in vitro*. For both conditions, the fresh weight of the aerial part (A and C) and the length of the primary root (B and D) were measured. Values represent mean ± SE (n ≥ 100). (*) indicates significant differences to the wild type plant control (WT; T-test, P < 0.05).

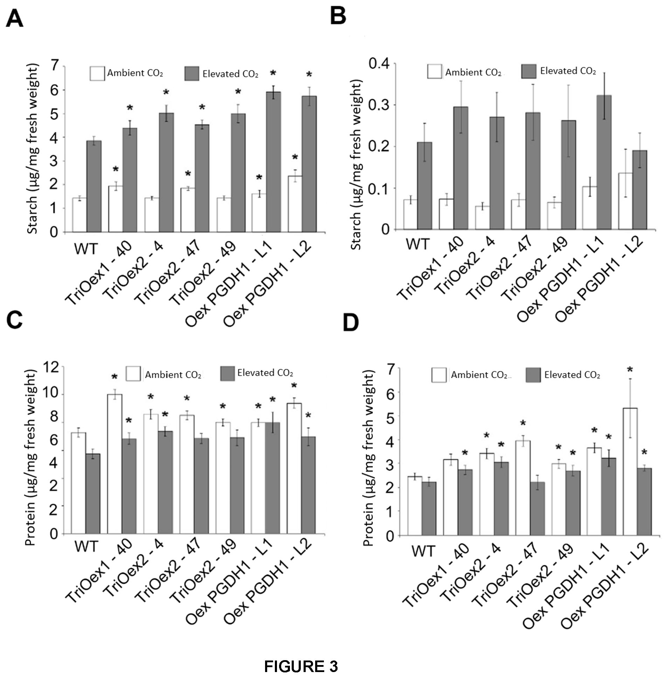

**Figure 3****.** Analysis of starch (A and B) and protein (C and D) content under ambient CO₂ (400 ppm; white bars) and elevated CO₂ (2500 ppm; grey bars) conditions in the aerial part (A and C) and in the roots (B and D) for 14-day-old *Arabidopsis thaliana* TriOex and Oex PGDH1 plants grown *in vitro*. Values represent mean ± SE (n = 12 biological replicates). (*) represents significant differences to the wild type plant control (WT; T-test, P < 0.05).

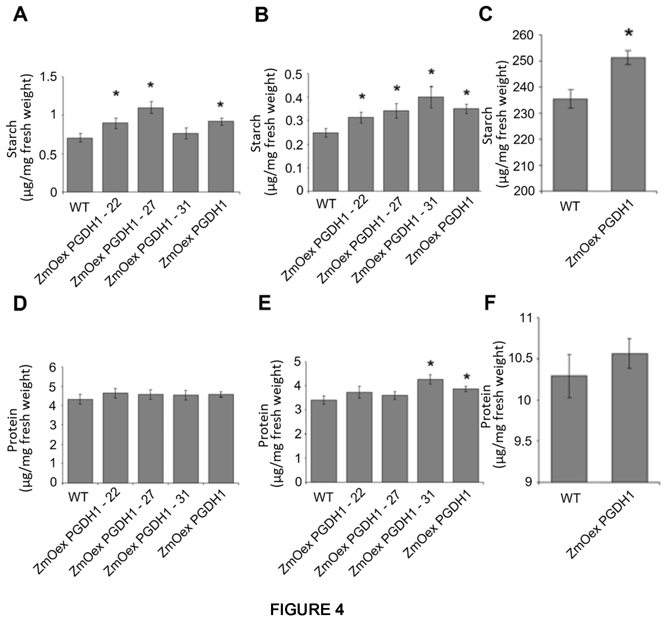

**Figure 4****.** Analysis of starch (A, B and C) and protein (D, E and F) content under ambient CO₂ conditions in leaves (A and D), stems (B and E) and in grain (C and F) of ZmOex PGDH1 corn plants either in individual lines (ZmOex PGDH1-22 and ZmOex PGDH1-27 and ZmOex PGDH1-31) or in all the lines as a whole (ZmOex PGDH1). The material comes from plants grown in greenhouses for 20 days (leaves and stems) or at the end of the biological cycle in the case of grain. Values represent mean ± SE (n ≥ 12 biological replicates). (*) represents significant differences to the wild type plant control (WT; T-test, P < 0.05).

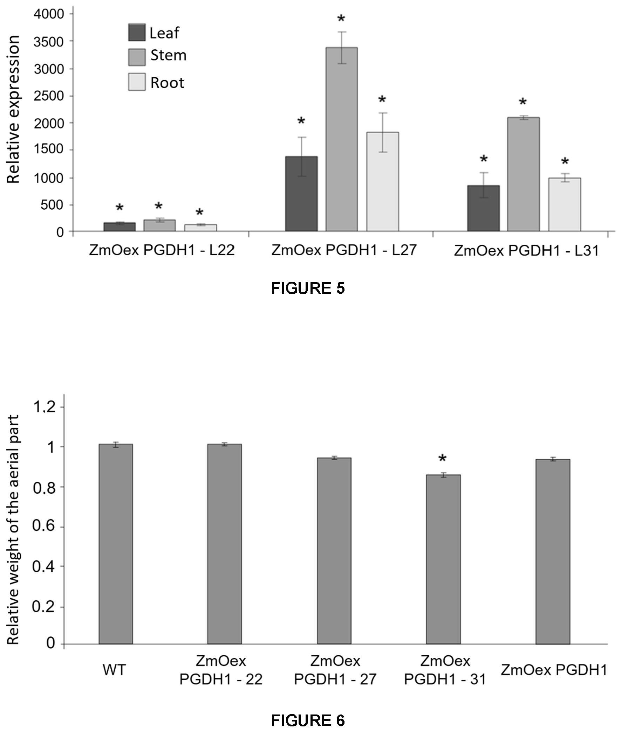

**Figure 5****.** Relative expression of the *Arabidopsis thaliana PGDH1* gene in different organs of three transgenic corn lines (ZmOex PGDH1-22 and ZmOex PGDH1-27 and ZmOex PGDH1-31). The bars represent mean ± SE (n ≥ 6 biological replicates). (*) represents significant differences to the wild type plant control (WT; T-test, P < 0.05).
**Figure 6****.** Relative weight of the aerial part of 22-day-old plants from three transgenic corn lines overexpressing the PGDH1 gene compared to the wild type control (WT). Data are either from individual transgenic lines (ZmOex PGDH1-22 and ZmOex PGDH1-27 and ZmOex PGDH1-31) or from all the lines as a whole (ZmOex PGDH1). The bars represent mean ± SE (n ≥ 15 biological replicates). (*) represents significant differences to the wild type plant control (WT; T-test, P < 0.05).
**Figure 7****.** Sequence alignment of *Arabidopsis thaliana* PGDH1 (AtPGDH1), *Arabidopsis thaliana* PGDH2 (AtPGDH2), *Arabidopsis thaliana* PGDH3 (*AtPGDH3*), *Homo sapiens* PGDH (HmPGDH), *Escherichia coli* PGDH (EcPGDH), rat PGDH1 (RnPGDH1), and *Mycobacterium tuberculosis* PGDH1 (MtPGDH1) proteins.

### DESCRIPTION OF EMBODIMENTS

### Example 1. Materials and methods

### Plant material and growing conditions

Wild type *Arabidopsis thaliana* ecotype Columbia plants were used as starting plant material. These wild type seeds were used as a genetic background to obtain overexpressing lines. The seeds to be sterilised were placed in a 1.5 mL tube and two successive washes of 15 minutes each were performed, under continuous agitation, with 1 mL of a solution of 70% ethanol and 0.05% SDS (Sodium Dodecyl Sulfate, SIGMA, Ref. 1667289). After these two washes, the final solution was removed and replaced with 1 mL of a 70% ethanol solution, again keeping the seeds agitated for 5 minutes. After these 5 minutes, the seeds were deposited and allowed to dry on sterile filter paper inside a horizontal laminar flow cabinet under sterile conditions. Once dry, the seeds were inoculated in the appropriate culture medium or stored at 4°C until use in a sterile 1.5 mL tube. For the *in vitro* culture of *Arabidopsis thaliana*, round or square plates of 12 cm on each side were used, containing a culture medium composed of 0.9 g/L of a preparation of Duchefa Biochemie (Ref. P03769-05), 0.9 g/L MES (2-(2-(N-morpholino)ethanesulfonic acid, SIGMA, Ref. 32K5465), 8 g/L agar (Pronadisa, Ref. SB090321), and the pH adjusted to 5.7 [adjusted with Tris (tris(hydroxymethyl) aminomethane; Sigma, Ref. T6066) or KOH]. This medium was designated as MS1/5. Once the inoculation was performed under sterile conditions, the plates were sealed with porous Micropore Hypoallergenic tape (3M Micropore TM, Ref. 1530-0 for the round plates and Ref. 1530-1 for the square plates), which allows the exchange of gases, but keeps the humidity inside the plates.

Before being transferred to growth chambers (Sanyo-MLR-351H or Ibercex H-900) for germination, the seeds inoculated on the plates were stratified 3-4 days at 4°C in order to favour and synchronise germination. The culture remained for the appropriate days for each experiment at a temperature of 22-25°C, relative humidity 40-60%, a long day photoperiod (16 h of light and 8 h of darkness) and a light intensity of 120 µmol·m⁻²·s⁻¹. When *in vitro* culture under elevated CO₂ conditions was necessary, plants were grown to a CO₂ concentration of 2000 or 2500 ppm under the same light and humidity conditions.

Plants of agronomic interest and with C₄ photosynthetic metabolism have also been used. Specifically, corn plants (*Zea mays*) variety B104 have been used to generate transgenic lines. This variety was supplied by the plant transformation service of Iowa State University where the first generation of transgenic corn plants was obtained. From this starting material, at least three more generations were obtained to achieve transgenic lines with a single insertion in homozygosity as indicated below.

The corn seeds were separated from the ear manually, cleaned and surface sterilised (to eliminate possible spores and fungi) by 2 successive 5-minute washes in alcoholic solutions. First, the seeds were placed in 250 mL flasks and sterilised under gentle agitation in a first wash with 70% ethanol with 0.05% SDS for 5 minutes. After 5 minutes this solution was removed and a second wash with 70% ethanol was performed for another 5 minutes. After this time, the seeds were deposited and allowed to dry on filter paper inside a laminar flow cabinet. Once the seeds were dry, they were stored at 4°C in paper bags until they were used.

To obtain material from corn leaves, stems and roots, their seeds were sown in polystyrene pots containing expanded perlite with a particle size of between 1-5 mm (Projar), placed in trays and watered with nutrient solution. After one week, the seeds had germinated and the seedlings had about 2 or 3 leaves. The material of leaves, stems and roots was collected at two weeks. It was necessary for the plants to complete their biological cycle to collect the grain. To do this, two-week-old seedlings were transplanted into 35-litre pots with a composition of Kekkila substrate and perlite in a 3:1 ratio (v/v), respectively.

The pots were watered 1 to 2 times per week with nutrient solution according to water needs. After a few months, the plants developed male inflorescences (from which we collected pollen) and female inflorescences (which self-pollinate) in order to obtain grain. When a small unopened ear (female inflorescence) appeared from the axil of a leaf, it was quickly covered with a paper bag to avoid unwanted crosses. Since in some cases there is a slight time lag between the development of the male flower, which develops earlier, and the female flower, the pollen was collected from each plant individually and stored at 4°C until use. At the time when the female flower developed and the filamentous stigmas appeared, each flower was pollinated with the pollen of the same plant, dropping it on the stigmas. The self-pollinations of the plants were prolonged during the period in which the male inflorescence generated pollen or while the stigmata were receptive.

The growth conditions in the greenhouse were long day (16 h of light and 8 h of dark), with an approximate light intensity of 130 µmol·m⁻²·s⁻¹, 50-70% relative humidity and a temperature around 28°C during the day and around 25°C during the night. Natural light was supplemented with artificial light (sodium vapour lamps and mercury vapour lamps) when necessary.

### Plant cloning and transformation

The *PGDH1* cDNA was introduced into the plant expression plasmid pMDC83, using Gateway^{®} technology, which is based on att recombination sites, using the recombinase enzyme from the INVITROGEN Gateway^{®} LR Clonase^{®} II enzyme mix kit (Ref. 11791-100). Plasmid pMDC83 allows expression in plants of *PGDH1* under the control of the strong cauliflower mosaic virus promoter (pMDC83 Pro35S:PGDH1-GFP). Plasmid pMDC83 is a carrier of the GFP green fusion protein that is placed in reading frame with the introduced DNA.

Triple overexpression plants of three genes encoding the three enzymes involved in the phosphorylation pathway of serine biosynthesis, D-3-phosphoglycerate dehydrogenase (PGDH1), phosphoserine aminotransferase (PSAT1) and phosphoserine phosphatase (PSP) have also been obtained. The construct to obtain plants overexpressing the *PSAT1* gene consisted of cloning the coding part of said gene in plasmid pMDC86 to obtain plasmid pMDC86 Pro35S:PSAT1-GFP. Plasmid pMDC86 is similar to pMDC83 described above except for the plant selection agent, which in plasmid pMDC83 provides tolerance to hygromycin and in plasmid pMDC86 provides tolerance to BASTA. The *PSAT1* gene does not contain introns, so BAC F8D20 was used to be able to PCR amplify its coding region. The resulting PCR product, using primers PSAT1+1 For and PSAT1+1270 Rev (**Table 4**), was cloned in the pCR8/GW/TOPO plasmid of the INVITROGEN pCR^{®}8/GW/TOPO^{®} TA Cloning Kit (Ref. K2500-20), with said plasmid subsequently transforming competent cells of *Escherichia coli* DH5α.

**Table 4** shows the primers used in the examples of the invention.

**Table 4**

| **Primer name** | **Sequence number** |
|---|---|
| PSAT1+1 For | SEQ ID NO: 10 |
| PSAT1+1270 Rev | SEQ ID NO: 11 |
| 2x35S -786 For | SEQ ID NO: 12 |
| T-Nos +322 *Bgl*II Rev | SEQ ID NO: 13 |
| EDA9GFP XbaI AvrII For | SEQ ID NO: 14 |
| EDA9GFP SpeI Rev | SEQ ID NO: 15 |

After checking by sequencing that the sequence of interest had no mutations, this coding region was subcloned into plasmid pMDC86, using Gateway^{®} technology. Plasmid pMDC86 is a carrier of the green GFP fusion protein that is placed in reading frame with the introduced DNA. The generated plasmid pMDC86 Pro35S:PSAT1-GFP was also checked by sequencing, but in this case only the insertion regions to confirm that the GFP was in reading phase with the coding region of the *PSAT1* gene. Finally, this construct was introduced into *Agrobacterium tumefaciens* strain GV3101 to transform *Arabidopsis thaliana* plants.

On the other hand, the construct of the *PSP1* gene was designed under the control of the *35S* promoter and linked to the gene encoding the GFP protein in plasmid pMDC100. Plasmid pMDC100 was chosen to obtain this construct due to providing tolerance in plants to kanamycin, and despite having recombination sites of the Gateway^{®} system, cloning of the sequences therein was by restriction enzymes. The *Pro35S:PSP1-GFP* fragment was obtained by PCR amplification using primers 2x35S -786 For and T-Nos +322 *Bgl*II Rev (**Table 4**) from construct pMDC83 *Pro35S:PSP1-GFP*. After verifying by means of electrophoresis with an aliquot of the PCR that the amplified fragment had the right size and there were no non-specific bands, the rest of the PCR reaction was purified and digested with the restriction enzyme *Hind*III, generating an amplicon with a sticky *Hind*III 5' end and a blunt 3' end. On the other hand, plasmid pMDC100 was digested with *Hind*III and *Ecl*136II (which generates a blunt end). In this way, by purifying the correct band, separated on an electrophoresis gel, the entire region between the attR1 and attR2 recombination regions was removed in pMDC100 and replaced by the *Pro35S:PSP1-GFP* fragment. In this way, plasmid pMDC100 was obtained, which confers resistance to kanamycin in plants and carries the *PSP1* gene under the control of the *35S* promoter and linked to the GFP (pMDC100 *Pro35S:PSP1-GFP*). After confirming that the insertion edges were correct by sequencing, this plasmid was introduced into *A. tumefaciens* strain GV3101 to transform *Arabidopsis thaliana* plants.

*Arabidopsis thaliana* plants were transformed using the floral immersion method. Several transgenic lines were generated independent of individuals homozygous for the transgene and with a single insertion.

To obtain the triple overexpression lines, it was started from plants overexpressing the *PGDH1* gene whose selection agent is the antibiotic hygromycin resistance gene. Two constructs were designed, as discussed above, to overexpress the genes encoding the enzyme phosphoserine aminotransferase (PSAT) and the enzyme phosphoserine phosphatase (PSP) in *Arabidopsis thaliana*. These two enzymes participate in the second and third reactions of the phosphorylation pathway of serine biosynthesis, respectively, while the PGDH1 enzyme participates in the first. The two constructs obtained for this purpose have been designed with two different plasmids, wherein each has a different selection agent (BASTA for the plasmid expressing the transgene containing *PSAT1* and kanamycin for the plasmid expressing the *PSP1* transgene) and also, in turn, different from the selection agent of the *PGDH1* overexpression line*s* (hygromycin). The presence of different selection agents in each of the overexpression lines made it easier to obtain the triple overexpression lines by being able to use antibiotic selection during seed germination.

With each of these constructs, two overexpression lines of the PGDH1 gene named *as* Oex PGDH1-L1 and Oex PGDH1-L2 were transformed. With this strategy, and for each of the Oex PGDH1 lines, plants overexpressing the *PSAT1* and *PGDH1* gene were obtained on the one *hand*, and on the other, plants overexpressing *PSP1* and *PGDH1*. Crosses were made from homozygous double overexpression plants until overexpression plants homozygous for the three transgenes were obtained. The identification of these lines homozygous for each of the three genes was possible to the different selection agents used.

A construction was also made to be able to express the cDNA *of Arabidopsis thaliana* PGDH1 in corn. This construct consisted of cloning the coding part of the *PGDH1* gene, linked to the GFP protein sequence, in plasmid pMCG1005 (obtained from Iowa State University). The *PGDH1-GFP* fragment was obtained from plasmid pMDC83 *Pro35S:PGDH1-GFP* by means of PCR using primers EDA9GFP XbaI AvrII For and EDA9GFP SpeI Rev (**Table 4**). After verifying by electrophoresis that the fragment had an approximate size of 2656 bp, the amplicon was purified and digested with the *Spe*I restriction enzyme to generate a sticky *Spe*I fragment on one side and blunt on the other. On the other hand, plasmid pMCG1005 was digested with *Spe*I and *Stu*I (the latter enzyme generates a blunt end) to remove the Waxy-a rice intron. After purifying the fragment corresponding to the plasmid (13220 bp) it was ligated with the *PGDH1-GFP* fragment to obtain the construct pMCG1005 *ProUbi:PGDH1-GFP*. This formed plasmid confers resistance to BASTA in plants and, after confirming that the sequence introduced into the plasmid was correct by sequencing, said plasmid was sent to the plant transformation service of Iowa State University to obtain transformed corn plants and send them to us.

Corn seeds obtained from plants transformed by the gene construct pMCG1005 *ProUbi:PGDH1-GFP,* from the plant transformation service of Iowa State University, were received and the plants that had a single copy of the transgene were identified and isolated. These seeds sent came from the transgenic plants regenerated after transformation, so according to the terminology normally used in *Arabidopsis thaliana*, they would correspond to the T2 segregating generation (where the lines that have a copy of the transgene are identified by a 3:1 ratio).

T2 seeds were sown for each independent line and after germination, BASTA tolerance assays and diagnostic PCRs were performed to identify the transgenic plants and the syngeneic controls. With the results it was possible to establish the segregation and, therefore, how many T-DNA insertions the parent plants had. After this assay, those lines of an insert were identified and within them the plants that were tolerant to BASTA and positive for PCR (and therefore contain the transgene of interest; these plants could already be homozygous or heterozygous but by the assays carried out we could not distinguish them). These plants selected after growing and flowering were self-pollinating to obtain the next generation of seeds. Once these seeds were obtained, they were re-sown and analysed to identify the batches of homozygous seeds (which would be 100% tolerant or PCR+ due to being their homozygous parent).

Since the life cycle of corn is between 6 and 7 months, the data shown are from some heterozygous lines and in some cases from homozygous lines. In the grain analyses, where there were not enough seeds, pools of two or three lines were made. *PGDH1* gene overexpression lines were named ZmOex PGDH1.

### Measurements of protein and starch content

Total soluble protein content was performed with Bradford Reagent (Bio-Rad, 500-0006). The starch content was determined with the Enzytec kit (r-biopharm). Two-week-old *Arabidopsis thaliana* rosette leaves were used in both cases. In the case of corn, leaves, stems or seeds were used and the protocol was optimised for each material.

### Example 2. Characterisation of Arabidopsis thaliana plants overexpressing the Arabidopsis thaliana PGDH1 gene

The content of free amino acids, starch and proteins in the overexpression lines of the *Arabidopsis thaliana* PGDH1 protein was analysed both under ambient CO₂ conditions and under elevated CO₂ conditions (2500 ppms of CO₂) where the activity of the glycolate pathway associated with photorespiratory activity is lower. Lines overexpressing the *PGDH1* gene (Oex PGDH1-L1 and Oex PGDH1-L2) and lines overexpressing the three pathway enzymes PGDH1, PSAT1 and PSP (TriOex1-40, TriOex2-4, TriOex2-47 and TriOex2-49) were used.

First, it was studied whether there was a negative effect of overexpression of the *Arabidopsis thaliana* PGDH1 protein on plant growth. In four of the six overexpression lines grown *in vitro* and cultured under ambient CO₂ conditions, no significant differences were observed in the growth of the aerial part with respect to the wild type plant control line (WT), and only the triple overexpression line TriOex2-49 and the Oex PGDH1-L2 line from which it is derived exhibited a lower growth of the aerial part (**Figure 2A**). A reduction in the main root growth (between 10 and 20%) was also observed in three of the six characterised lines (TriOex2-4, TriOex2-49 and the Oex PGDH1-L2 line from which they are derived (**Figure 2B**).

*In vitro* phenotypic characterisation assays were also performed under elevated CO₂ conditions, to study the role of the *Arabidopsis thaliana* PGDH1 protein when the glycolate pathway associated with photorespiration has a lower activity. No significant differences in aerial part growth were observed in this trial in any of the TriOex lines compared to control. Only a decrease in weight of the aerial part of around 25% was observed in the Oex PGDH1-L2 line (**Figure 2C**). Contrary to what occurred under ambient CO₂ conditions, a significant increase in the length of the primary root in three lines was observed when compared to the control (TriOex1-40, TriOex2-47 and TriOex2-49) with an increase between 7-12% of the primary root compared to the control (**Figure 2D**).

With these results it is concluded that it is possible to obtain overexpression lines of the *Arabidopsis thaliana* PGDH1 protein without having a negative effect on the growth of *Arabidopsis thaliana* and that overexpression can even have a positive effect on growth under culture conditions with elevated CO₂.

The metabolomic analysis of the overexpression lines of the *Arabidopsis thaliana* PGDH1 protein showed an increase in the serine content accompanied by the generalised increase in other amino acids in most of the overexpression lines, mainly in the aerial part but also in roots, among which threonine, valine, asparagine, aspartate, glutamate and glutamine should be highlighted (**Table 5** and **Table 6**) suggesting that serine levels are essential to control the homeostasis of the rest of amino acids in the plant, and therefore of the processes associated with it, fundamentally protein synthesis. Of all of them, the most interesting are valine and threonine because they are essential amino acids, but above all threonine because it is considered one of the four essential amino acids that limit the nutritional quality of plants.

**Tables 5 and 6** show the levels of amino acids in the aerial part (Table 5) and in roots (Table 6) of the overexpression lines of the PGDH1 gene (OexPGDH1-L1 and OexPGDH1-L2) and the triple overexpression lines (TriOex1-40, TriOex2-4, TriOex2-47 and TriOex2-49) compared to the wild type plant line (WT) under ambient CO₂ conditions (aCO₂; 400 ppm). The data represent normalised relative values with respect to the mean calculated for the WT (mean ± SE of at least 6 independent determinations). Values that are significantly different from the wild type control (WT) are highlighted in bold (T-test, P < 0.05).

**Table 5**

| **Amino acids** | **WT aCO₂** | **Oex PGDH1 L1 aCO₂** | **Oex PGDH1 L2 aCO₂** | **TriOex1-40 aCO₂** | **TriOex2-4 aCO₂** | **TriOex2-47 aCoO₂** | **TriOex2-49 aCO₂** |
|---|---|---|---|---|---|---|---|
| Aspartic acid | 1±0.06 | **1.41±0.13** | **1.65±0.06** | **1.27±0.07** | **1.30±0.08** | **1.36±0.11** | **1.39±0.15** |
| Alanine | 1±0.11 | 0.80±0.13 | 1.06±0.19 | 1.00±0.16 | 0.84±0.13 | 0.91±0.17 | 0.90±0.18 |
| Alanine, β | 1±0.03 | **1.18±0.07** | **1.34±0.02** | **1.31±0.08** | **1.26±0.06** | 1.15±0.10 | 1.31±0.14 |
| Asparagine | 1±0.19 | **2.00±0.40** | **2.62±0.70** | 1.66±0.32 | **1.89±0.34** | 1.94±0.43 | 1.55±0.31 |
| Phenylalanine | 1±0.03 | 1.08±0.07 | 1.04±0.03 | **1.17±0.05** | **1.12±0.03** | 1.02±0.05 | 1.05±0.09 |
| Glycine | 1±0.04 | **1.43±0.08** | **1.65±0.13** | **1.33±0.13** | 1.11±0.05 | 1.12±0.06 | 0.88±0.06 |
| Glutamate | 1±0.08 | **1.86±0.23** | **1.71±0.26** | **1.50±0.18** | **1.82±0.25** | **1.64±0.26** | 1.34±0.21 |
| Glutamine | 1±0.13 | **2.14±0.22** | **2.03±0.14** | **1.96±0.37** | **2.47±0.12** | **1.99±0.40** | 1.45±0.24 |
| Homoserine | 1±0.05 | 1.16±0.10 | 1.14±0.07 | 1.17±0.07 | 1.11±0.05 | 1.10±0.05 | 0.94±0.04 |
| Isoleucine | 1±0.03 | **1.15±0.05** | **1.13±0.03** | **1.18±0.07** | 1.09±0.05 | 0.97±0.04 | 1.09±0.07 |
| Leucine | 1±0.04 | 1.16±0.08 | **1.18±0.02** | 1.12±0.08 | 1.06±0.05 | 0.99±0.05 | 1.07±0.08 |
| Lysine | 1±0.08 | 1.20±0.09 | 1.01±0.08 | 1.15±0.12 | **1.32±0.07** | **1.29±0.06** | 1.13±0.11 |
| Methionine | 1±0.04 | 1.14±0.11 | 0.98±0.04 | **1.19±0.06** | 1.23±0.12 | **1.30±0.12** | 1.17±0.14 |
| Ornithine | 1±0.08 | 1.27±0.13 | 1.14±0.16 | 1.03±0.07 | 1.20±0.05 | **1.44±0.13** | 0.88±0.07 |
| Proline | 1±0.06 | 1.14±0.12 | 1.20±0.09 | 1.26±0.11 | 1.04±0.07 | **0.81±0.05** | 0.99±0.09 |
| Serine | 1±0.07 | **2.14±0.19** | **2.38±0.18** | **1.56±0.15** | **1.99±0.18** | **2.03±0.15** | **1.90±0.21** |
| Threonine | 1±0.03 | **1.40±0.08** | **1.46±0.04** | **1.33±0.08** | **1.44±0.07** | **1.32±0.06** | **1.33±0.11** |
| Valine | 1±0.02 | **1.18±0.05** | **1.28±0.02** | **1.20±0.07** | **1.17±0.04** | 1.04±0.08 | 1.08±0.06 |

**Table 6**

| **Amino acids** | **WT aCO₂** | **OexPGDH1 L1 aCO₂** | **OexPGDH1 L2 aCO₂** | **TriOex1-40 aCO₂** | **TriOex2-4 aCO₂** | **TriOex2-47 aCO₂** | **TriOex2-49 aCO₂** |
|---|---|---|---|---|---|---|---|
| Aspartic acid | 1±0.08 | **1.26±0.08** | **1.23±0.03** | 1.05±0.04 | 1.17±0.04 | **1.31±0.04** | 1.15±0.05 |
| Alanine | 1±0.10 | 0.90±0.12 | 1.12±0.22 | 1.14±0.17 | 1.04±0.15 | 1.01±0.16 | 1.08±0.16 |
| Alanine, β | 1±0.08 | 1.16±0.07 | 1.19±0.08 | 1.07±0.06 | 1.23±0.08 | 1.23±0.07 | 1.01±0.08 |
| Asparagine | 1±0.10 | **1.80±0.23** | **1.77±0.19** | 1.32±0.10 | **1.85±0.17** | **2.01±0.24** | 1.59±0.25 |
| Phenylalanine | 1±0.09 | **1.48±0.07** | **1.29±0.02** | 1.16±0.06 | **1.58±0.05** | **1.69±0.06** | **1.49±0.07** |
| Glycine | 1±0.03 | **1.59±0.06** | **1.67±0.05** | 1.07±0.06 | **1.51±0.04** | **1.77±0.08** | **1.32±0.05** |
| Glutamate | 1±0.17 | **1.61±0.19** | **1.57±0.17** | 1.16±0.09 | 1.48±0.14 | **1.79±0.13** | 1.41±0.17 |
| Glutamine | 1±0.13 | **1.82±0.15** | **2.09±0.1** | 1.26±0.10 | **1.70±0.10** | **2.06±0.16** | 1.41±0.19 |
| Homoserine | 1±0.04 | **1.32±0.06** | **1.29±0.06** | 1.16±0.08 | **1.17±0.04** | **1.43±0.03** | **1.19±0.06** |
| Isoleucine | 1±0.04 | 1.02±0.02 | 0.92±0.02 | 1.04±0.04 | 0.98±0.01 | 1.10±0.03 | 0.99±0.03 |
| Leucine | 1±0.04 | **1.12±0.02** | 1.04±0.02 | 1.09±0.04 | 1.09±0.02 | **1.15±0.03** | 1.02±0.02 |
| Lysine | 1±0.07 | 0.97±0.02 | 0.84±0.03 | 0.88±0.05 | 0.95±0.04 | 1.12±0.05 | 0.88±0.04 |
| Methionine | 1±0.07 | 1.05±0.04 | 1.16±0.07 | 1.07±0.09 | 1.06±0.06 | **1.33±0.09** | 1.19±0.07 |
| Ornithine | 1±0.12 | 1.01±0.08 | 0.95±0.08 | 1.00±0.07 | 1.01±0.06 | 1.31±0.10 | 1.09±0.12 |
| Proline | 1±0.05 | 1.02±0.08 | 1.02±0.02 | 0.98±0.03 | 1.06±0.05 | 0.95±0.04 | **0.87±0.02** |
| Serine | 1±0.05 | **5.43±0.18** | **5.06±0.10** | **1.49±0.08** | **4.74±0.13** | **5.99±0.23** | **4.50±0.21** |
| Threonine | 1±0.05 | **1.58±0.07** | **1.54±0.03** | **1.19±0.06** | **1.69±0.03** | **1.87±0.05** | **1.54±0.08** |
| Valine | 1±0.04 | **1.19±0.03** | **1.10±0.01** | 1.09±0.04 | **1.17±0.02** | **1.25±0.03** | **1.11±0.03** |

Metabolomic analysis was also performed on plants grown in elevated CO₂ concentrations. In this case, a generalised increase was observed of practically all amino acids in the aerial part of the overexpression lines, with special attention to the essential amino acids isoleucine, leucine, lysine, phenylalanine, methionine and valine (**Table 7**). In roots, an increase in phenylalanine and threonine was also observed (**Table 8**).

**Tables 7 and 8** show the levels of amino acids in the aerial part (**Table 7**) and in roots (**Table 8**) of the PGDH1 gene overexpression lines (OexPGDH1-L1 and OexPGDH1-L2) and the triple overexpression lines (TriOex1-40, TriOex2-4, TriOex2-47 and TriOex2-49) compared to the wild type plant line (WT) under elevated CO₂ conditions (eCO₂; 2500 ppm). The data represent normalised relative values with respect to the mean calculated for wild type plants (WT) (mean ± SE of at least 6 independent determinations). Values that are significantly different to wild type (WT) are highlighted in bold (T-test, P < 0.05).

**Table 7**

| **Amino acids** | **WT eCO₂** | **Oex PGDH1 L1 eCO₂** | **OexPGDH1 L2 eCO₂** | **TriOex1-40 eCO₂** | **TriOex2-4 eCO₂** | **TriOex2-47 eCO₂** | **TriOex2-49 eCO₂** |
|---|---|---|---|---|---|---|---|
| Aspartic acid | 1±0.02 | **1.74±0.08** | **1.62±0.07** | **1.33±0.03** | **1.45±0.21** | **1.59±0.08** | **1.62±0.07** |
| Alanine | 1±0.11 | 0.74±0.16 | 0.80±0.12 | 0.92±0.11 | **0.63±0.09** | 0.99±0.21 | 1.14±0.21 |
| Alanine, β | 1±0.09 | **1.47±0.10** | **1.65±0.07** | **1.42±0.13** | **1.77±0.11** | **1.75±0.22** | **1.67±0.18** |
| Asparagine | 1±0.12 | **2.10±0.27** | **1.93±0.24** | 1.73±0.35 | **2.91±0.64** | **2.27±0.41** | 1.70±0.34 |
| Phenylalanine | 1±0.05 | **1.35±0.13** | **1.73±0.09** | **1.51±0.05** | **2.31±0.36** | **1.98±0.17** | **1.53±0.08** |
| Glycine | 1±0.05 | **1.46±0.11** | **1.77±0.10** | **1.50±0.14** | **1.66±0.1** | **1.79±0.11** | **1.43±0.16** |
| Glutamate | 1±0.05 | **1.85±0.12** | **2.11±0.12** | **1.70±0.17** | **2.54±0.07** | **2.20±0.15** | **1.72±0.20** |
| Glutamine | 1±0.13 | **1.91±0.16** | **1.61±0.12** | **1.41±0.10** | **2.81±0.21** | **1.79±0.11** | 1.22±0.18 |
| Homoserine | 1±0.05 | **1.30±0.09** | **1.62±0.11** | **1.52±0.18** | **1.70±0.15** | **1.89±0.17** | 1.29±0.19 |
| Isoleucine | 1±0.03 | **1.51±0.09** | **1.52±0.03** | **1.39±0.07** | **1.94±0.11** | **1.65±0.14** | **1.68±0.11** |
| Leucine | 1±0.03 | **1.51±0.10** | **1.53±0.03** | **1.37±0.05** | **2.01±0.15** | **1.71±0.16** | **1.78±0.13** |
| Lysine | 1±0.06 | **1.37±0.14** | **1.98±0.16** | **1.85±0.26** | **2.60±0.20** | **2.55±0.33** | **1.95±0.23** |
| Methionine | 1±0.12 | 1.11±0.15 | **2.03±0.31** | **2.08±0.29** | 1.54±0.41 | **1.89±0.36** | **1.91±0.39** |
| Ornithine | 1±0.06 | 1.18±0.12 | **1.90±0.31** | **1.97±0.29** | **2.73±0.24** | **3.08±0.27** | **1.69±0.28** |
| Proline | 1±0.02 | **1.83±0.14** | **1.38±0.06** | **1.22±0.05** | **2.02±0.54** | **1.26±0.08** | **1.68±0.23** |
| Serine | 1±0.03 | **2.53±0.12** | **2.81±0.06** | **1.61±0.10** | **2.78±0.10** | **3.11±0.13** | **2.80±0.25** |
| Threonine | 1±0.02 | **1.79±0.11** | **2.02±0.04** | **1.57±0.08** | **2.16±0.08** | **2.21±0.09** | **2.01±0.15** |
| Valine | 1±0.03 | **1.59±0.08** | **1.58±0.04** | **1.33±0.05** | **1.85±0.10** | **1.66±0.12** | **1.57±0.09** |

**Table 8**

| **Amino acids** | **WT eCO₂** | **OexPGDH1 L1 eCO₂** | **OexPGDH1 L2 eCO₂** | **TriOex1-40 eCO₂** | **TriOex2-4 eCO₂** | **TriOex2-47 eCO₂** | **TriOex2-49 eCO₂** |
|---|---|---|---|---|---|---|---|
| Aspartic acid | 1±0.04 | **1.39±0.04** | 1.03±0.03 | 1.01±0.06 | **1.68±0.06** | **1.30±0.06** | 1.12±0.07 |
| Alanine | 1±0.15 | 1.40±0.19 | 0.93±0.16 | 0.81±0.14 | 1.05±0.15 | 0.83±0.13 | 0.96±0.22 |
| Alanine, β | 1±0.02 | **1.71±0.07** | **1.38±0.06** | **1.16±0.05** | **1.90±0.07** | **1.57±0.07** | **1.34±0.05** |
| Asparagine | 1±0.03 | **1.77±0.13** | 1.15±0.09 | 1.06±0.12 | **2.51±0.27** | **1.60±0.17** | **1.26±0.08** |
| Phenylalanine | 1±0.06 | **1.93±0.10** | **1.43±0.04** | **1.27±0.04** | **2.33±0.15** | **1.71±0.06** | **1.33±0.09** |
| Glycine | 1±0.02 | **1.59±0.04** | **1.41±0.04** | **1.18±0.03** | **1.84±0.06** | **1.73±0.05** | **1.34±0.06** |
| Glutamate | 1±0.03 | **1.65±0.08** | **1.17±0.06** | 1.02±0.05 | **2.35±0.13** | **1.61±0.11** | **1.32±0.09** |
| Glutamine | 1±0.05 | **1.91±0.09** | **1.34±0.08** | 1.12±0.05 | **2.78±0.15** | **1.77±0.09** | **1.44±0.10** |
| Homoserine | 1±0.02 | **1.44±0.02** | **1.15±0.04** | 1.08±0.06 | **1.56±0.06** | **1.38±0.04** | 1.03±0.14 |
| Isoleucine | 1±0.07 | 1.06±0.02 | 0.86±0.04 | 1.01±0.02 | **1.43±0.03** | 1.15±0.04 | 0.93±0.09 |
| Leucine | 1±0.06 | **1.16±0.03** | 0.95±0.05 | 1.07±0.02 | **1.62±0.05** | **1.30±0.04** | 1.02±0.08 |
| Lysine | 1±0.05 | **1.30±0.05** | 0.98±0.05 | 1.16±0.09 | **1.81±0.07** | **1.50±0.07** | 0.99±0.14 |
| Methionine | 1±0.07 | 1.12±0.06 | 1.18±0.06 | 1.06±0.09 | **1.42±0.09** | **1.27±0.10** | 1.03±0.09 |
| Ornithine | 1±0.11 | 1.06±0.05 | 0.81±0.05 | 1.03±0.07 | **1.81±0.06** | **1.49±0.10** | 0.96±0.16 |
| Proline | 1±0.04 | **1.84±0.08** | 0.93±0.04 | 0.97±0.02 | **1.65±0.07** | **1.17±0.04** | 1.10±0.07 |
| Serine | 1±0.04 | **4.58±0.12** | **3.43±0.10** | **1.37±0.05** | **4.37±0.11** | **4.54±0.15** | **3.47±0.15** |
| Threonine | 1±0.06 | **1.65±0.03** | **1.23±0.06** | 1.11±0.04 | **2.18±0.06** | **1.63±0.05** | **1.35±0.10** |
| Valine | 1±0.06 | **1.23±0.02** | 0.96±0.04 | 1.03±0.03 | **1.58±0.04** | **1.25±0.03** | 1.02±0.08 |

These results indicate that the effect of overexpression of the *Arabidopsis thaliana* PGDH1 protein on amino acid content is especially relevant in elevated CO₂ conditions in the aerial part, since in these conditions the levels of certain amino acids, such as the essential amino acids isoleucine, leucine, lysine, methionine, phenylalanine, valine or threonine are reduced in control plants compared to environmental CO₂ conditions (**Table 9**).

**Table 9** shows the amino acid levels in the aerial part of the control line (WT), the PGDH1 gene overexpression lines (OexPGDH1-L1 and OexPGDH1-L2) and the triple overexpression lines (TriOex1-40, TriOex2-4, TriOex2-47 and TriOex2-49) under elevated CO₂ conditions (eCO₂; 2500 ppm) compared to the wild type plant (WT) grown under ambient CO₂ conditions (aCO₂; 400 ppm). The data represent normalised relative values with respect to the mean calculated for the wild type plant (WT) in environmental CO₂ (mean ± SE of at least 6 independent determinations). Values that are significantly different to wild type (WT) are highlighted in bold (T-test, P < 0.05).

**Table 9**

| **Amino acids** | **WT aCO₂** | **WT eCO₂** | **OexPGDH 1 L1 eCO₂** | **OexPGDH 1 L2 eCO₂** | **TriOex1 -40 eCO₂** | **TriOex2-4 eCO₂** | **TriOex2-47 eCO₂** | **TriOex2 -49 eCO₂** |
|---|---|---|---|---|---|---|---|---|
| Aspartic acid | 1± 0.06 | **1.19± 0.03** | **2.06±0.09** | **1.92±0.08** | **1.58± 0.04** | **1.72±0.26** | **1.88±0.10** | **1.92± 0.08** |
| Alanine | 1± 0.11 | 0.75± 0.08 | **0.55±0.12** | **0.60±0.09** | 0.69± 0.08 | **0.47±0.07** | 0.74±0.16 | 0.85± 0.16 |
| Alanine, β | 1± 0.03 | 0.85± 0.07 | **1.26±0.08** | **1.41±0.06** | 1.21± 0.11 | **1.51±0.10** | **1.49±0.19** | **1.42± 0.15** |
| Asparagine | 1± 0.19 | **1.74± 0.20** | **3.65±0.48** | **3.35±0.42** | **3.01± 0.62** | **5.05±1.12** | **3.94±0.71** | **2.96± 0.59** |
| Phenylalanin e | 1± 0.03 | **0.70± 0.04** | 0.94±0.09 | **1.21±0.07** | 1.06± 0.03 | **1.61±0.25** | **1.38±0.12** | 1.07± 0.05 |
| Glycine | 1± 0.04 | **0.17± 0.01** | **0.25±0.02** | **0.31±0.02** | **0.26± 0.02** | **0.29±0.02** | **0.31±0.02** | **0.25± 0.03** |
| Glutamate | 1± 0.08 | **1.19± 0.06** | **2.19±0.14** | **2.50±0.14** | **2.02± 0.21** | **3.02±0.09** | **2.61±0.18** | **2.05± 0.23** |
| Glutamine | 1± 0.13 | **6.92± 0.88** | **13.22±1.11** | **9.73±1.55** | **9.73± 0.70** | **19.43±1.4 8** | **12.41±0.7 4** | **8.42± 1.22** |
| Homoserine | 1± 0.05 | **0.59± 0.03** | **0.76±0.05** | 0.95±0.07 | 0.89± 0.11 | 1.00±0.09 | 1.11±0.10 | 0.76± 0.11 |
| Isoleucine | 1± 0.03 | **0.55± 0.02** | **0.83±0.05** | **0.84±0.02** | **0.77± 0.04** | 1.07±0.06 | 0.91±0.08 | 0.93± 0.06 |
| Leucine | 1± 0.04 | **0.51± 0.02** | **0.78±0.05** | **0.78±0.02** | **0.70± 0.03** | 1.03±0.08 | 0.88±0.08 | 0.91± 0.06 |
| Lysine | 1± 0.08 | **0.42± 0.02** | **0.57±0.06** | 0.82±0.07 | 0.77± 0.11 | 0.91±0.18 | 1.06±0.14 | 0.81± 0.10 |
| Methionine | 1± 0.04 | 0.40± 0.05 | **0.44±0.06** | 0.80±0.12 | 0.83± 0.12 | **0.61±0.16** | 0.75±0.14 | 0.76± 0.15 |
| Ornithine | 1±0.0 8 | **0.39±0.0 2** | **0.46±0.05** | 0.74±0.12 | 0.77± 0.11 | 1.06±0.09 | 1.20±0.10 | **0.66± 0.11** |
| Proline | 1±0.0 6 | **0.24±0.0 1** | **0.45±0.03** | **0.34±0.01** | **0.30± 0.01** | **0.49±0.13** | **0.31±0.02** | **0.41± 0.06** |
| Serine | 1±0.0 7 | **0.52±0.0 1** | **1.31±0.06** | **1.46±0.03** | 0.83± 0.05 | **1.44±0.05** | **1.61±0.07** | **1.45± 0.13** |
| Threonine | 1±0.0 3 | **0.63±0.0 2** | 1.12±0.07 | **1.27±0.02** | 0.98± 0.05 | **1.36±0.05** | **1.39±0.06** | **1.26± 0.09** |
| Valine | 1±0.0 2 | **0.63±0.0 2** | 1.01±0.05 | 1.01±0.02 | **0.85± 0.03** | **1.17±0.06** | 1.05±0.07 | 1.00± 0.06 |

The starch and protein levels in the overexpression lines of the *Arabidopsis thaliana* PGDH1 protein were quantified under ambient CO₂ conditions and under elevated CO₂ conditions.

Under ambient CO₂ conditions, a significant increase in starch content was observed with respect to the control, in the aerial part of four of the overexpression lines analysed (TriOex2-47, TriOex1-40, Oex PGDH1-L1 and Oex PGDH1-L2) (**Figure 3A**). This increase could be observed in the aerial part of all overexpression lines when the plants were grown under elevated CO₂ conditions (**Figure 3A**). No significant differences in roots were observed between overexpression and control plants under any of the conditions tested (**Figure 3B**).

Under cultivation conditions in the presence of elevated CO₂ concentrations, plants synthesise and store more starch to the detriment of the protein content. This is partly because the Rubisco enzyme acts mostly with carboxylase activity so that all the D-3-phosphoglycerate formed enters the Calvin cycle to synthesise sugars. Both the control and overexpression plants of the *Arabidopsis thaliana* PGDH1 protein were found to have higher starch content under these conditions, but the overexpressors always showed higher levels than the controls (**Figure 3**). Moreover, all lines showed lower protein content under these elevated CO₂ conditions, but this reduction in protein content was less in overexpression lines than in both aerial and root controls (**Figure 3C** and **Figure 3D**). Thus, plants overexpressing the *Arabidopsis thaliana* PGDH1 protein had higher protein content than controls under both ambient CO₂ and elevated CO₂ conditions (**Figure 3C** and **Figure 3D**). In addition, under elevated CO₂ conditions, the protein content in the overexpression lines was similar or even higher than the control value under ambient CO₂ conditions (**Figure 3C** and **Figure 3D**).

The results obtained demonstrate that the stimulatory effect of the overexpression of the *Arabidopsis thaliana* PGDH1 protein on amino acid metabolism implies in turn an increase in the protein content in the *Arabidopsis thaliana* overexpression lines and, surprisingly, a higher starch content with respect to the control without affecting the growth of the plant. On the one hand, the increased serine content of the phosphorylation pathway of serine biosynthesis can stimulate the synthesis of the remaining amino acids that activate protein synthesis. Moreover, the higher serine content of the phosphorylation pathway of serine biosynthesis would imply that a lower content of the photorespiratory serine exits the cycle and is recycled back to D-3-phosphoglycerate, thereby increasing the assimilation of sugars and ultimately being stored in the form of starch.

The results obtained also show that the increase in atmospheric CO₂ levels affects carbon and nitrogen homeostasis, favouring carbohydrate metabolism and reducing that of amino acids and proteins. The effect of the phosphorylation pathway of serine biosynthesis on carbohydrate/protein homeostasis is most evident under conditions of climate change. The results obtained demonstrate that genetic engineering of the phosphorylation pathway of serine biosynthesis is extremely useful as a strategy to increase the nutritional quality of plants, especially under conditions of reduced photorespiratory activity associated with the increase in atmospheric CO₂.

### Example 3. Characterisation of corn plants overexpressing the Arabidopsis thaliana PGDH1 gene

The contribution of overexpression of the *Arabidopsis thaliana* PGDH1 protein was studied in a species with C₄ metabolism, such as corn, with a lower photorespiratory rate than *Arabidopsis thaliana* and, therefore, with less activity of the glycolate pathway. To this end, the starch and protein content of transgenic corn plants overexpressing the *Arabidopsis thaliana PGDH1* gene (*ZmOex* PGDH1) grown in greenhouses with ambient CO₂ concentrations was analysed. In this case, not only were results obtained from the leaves and roots, but also the contents in the stems of three ZmOex PGDH1 lines (ZmOex PGDH1-22, ZmOex PGDH1-27, and ZmOex PGDH1-31) were measured together with the control line (WT).

With respect to starch, in corn plants heterozygous for the PGDH1 transgene, two of the overexpression lines of the *Arabidopsis thaliana* PGDH1 protein (ZmOex PGDH1-22 and ZmOex PGDH1-27) significantly increased their content in both leaves and stems compared to the control (**Figure 4A** and **Figure 4B**). A third line also increased the starch content in stems (ZmOex PGDH1-31). This increase ranged between 8% and 61% depending on the lines indicating that there is high variability between lines. In order to give greater robustness to the statistical analysis, an analysis was conducted of all the overexpression lines as a whole (ZmOex PGDH1), and they were compared with the control lines. Thus, a significant increase in starch content was observed in the overexpression lines with respect to the controls, around 29% in leaves and 42% in stems.

Regarding proteins, in plants heterozygous for the *PGDH1* transgene, all lines showed a higher content than controls in leaves and stems, ranging between 5% and 25% with respect to the control, although it was only significant in one line in stems (**Figure 4D** and **Figure 4E**).

Therefore, after obtaining homozygous lines of corn for the *PGDH1* transgene*,* new experiments were performed and one line with a significant increase in the protein content in leaves and three lines with a significant increase in stems were observed (**Table 10**). The pooled analysis of all lines showed a significant increase between 9 and 18% of the protein content in leaves and stems respectively. **Table 10** shows the protein content in leaves and stems of corn plants overexpressing the *Arabidopsis thaliana PGDH1* gene. Data are either from individual lines (ZmOex PGDH1-22 and ZmOex PGDH1-27 and ZmOex PGDH1-31) or from of all the lines as a whole (ZmOex PGDH1). The material comes from plants grown in greenhouses for 15 days under ambient CO₂ conditions. The values represent the mean expressed as a decimal to the wild type plant control (WT) ± SE (n ≥ 30 biological replicates). (*) represents significant differences to the wild type control (WT; T-test, P < 0.05).

**Table 10**

| Lines | Relative content | | |
|---|---|---|---|
| | Leaves | Stems | Grain |
| WT | 1.00 ± 0.03 | 1.00 ± 0.03 | 1.00 ± 0.01 |
| ZmOex PGDH1-22 | 1.06 ± 0.03 | 1.14 ± 0.03* | 1.03 ± 0.02 |
| ZmOex PGDH1-27 | 1.07 ± 0.03 | 1.18 ± 0.05* | 1.02 ± 0.02 |
| ZmOex PGDH1-31 | 1.15 ± 0.04* | 1.23 ± 0.05* | 1.05 ± 0.02* |
| ZmOex PGDH1 | 1.09 ± 0.02* | 1.18 ± 0.02* | 1.03 ± 0.01 |

Finally, the starch and protein content in the corn grain of the lines overexpressing the *Arabidopsis thaliana* PGDH1 protein was determined. As in previous cases and to increase the robustness of the statistical analysis, the data of the three overexpression lines were compared as a whole. In the case of starch, a significant increase in starch content of 6.7% was observed. In proteins, only a 2.6% increase was observed, which was not significant (**Figure 4**). The higher percentages of increase in stems and leaves with respect to grain can be attributed in part to the fact that some of the mother plants were not homozygous, and therefore some of the grains from these lines will not be transgenic. Repeating these experiments obtained from grains of homozygous lines improved these results. Thus, the protein content also increased significantly in the grain in one of the lines studied by 5%, while in the other two the increase was between 2 and 3% (Table 10).

An analysis of the gene expression of the *PGDH1* gene in the transgenic lines indicated that the ZmOex PGDH1-27 and ZmOex PGDH1-31 lines show the highest level of expression of the transgene in all the organs studied (**Figure 5**).

In addition, the contribution of overexpression of the *Arabidopsis thaliana* PGDH1 protein under elevated CO₂ conditions in a species with C₄ photosynthetic metabolism, such as corn, was studied. Plants with C₄ photosynthetic metabolism in principle have a lower photorespiratory rate than plants with C₃ metabolism, but not zero, so it is important to know the effects of the *Arabidopsis thaliana* PGDH1 protein on this type of metabolism in both ambient and elevated CO₂. To this end, the protein content of transgenic corn plants overexpressing the PGDH1 gene in homozygosity of *Arabidopsis thaliana* (ZmOex PGDH1) was analysed. In this case, results were obtained on leaves and stems of three ZmOex PGDH1 lines (ZmOex PGDH1-22, ZmOex PGDH1-27 and ZmOex PGDH1-31) together with the wild type plant control (WT) line.

Regarding the protein content, all the lines showed a higher content than the leaf controls, being between 14% and 22% in the best case (**Table 11**). In stems, the lines with the highest level of PGDH1 expression also showed a significant increase in protein content, being between 17% and 38% higher than in the controls (**Table 11**). The analysis of all the overexpression lines as a whole (ZmOex PGDH1) with respect to the wild type plant control (WT) is also presented. In that case, the protein content of leaves and stems increased significantly by 18% and 20%, respectively, compared to the wild type plant control (WT) (**Table 11**). Data are either from individual lines (ZmOex PGDH1-22 and ZmOex PGDH1-27 and ZmOex PGDH1-31) or from all the lines as a whole (ZmOex PGDH1). The material comes from plants grown in greenhouses for 15 days in the presence of 2000 ppms of CO₂. The values represent the mean expressed as a decimal to the wild type plant control (WT) ± SE (n ≥ 30 biological replicates). (*) represents significant differences to the wild type plant control (WT; T-test, P < 0.05).

**Table 11**

| Lines | Relative content | |
|---|---|---|
| | Leaves | Stems |
| WT | 1.00 ± 0.02 | 1.00 ± 0.04 |
| ZmOex PGDH1-22 | 1.15 ± 0.02* | 1.04 ± 0.05 |
| ZmOex PGDH1-27 | 1.16 ± 0.04* | 1.17 ± 0.05* |
| ZmOex PGDH1-31 | 1.22 ± 0.03* | 1.39 ± 0.08* |
| ZmOex PGDH1 | 1.18 ± 0.02* | 1.20 ± 0.04* |

It is important to note that the increase in protein content occurred without significant reduction of aerial part growth in two of the overexpression lines as shown in **Figure 6**. Only the ZmOex PGDH1-31 line showed a growth reduction of 15%. But this reduction was less than the 22% and 39% increase in protein content in leaves and stems respectively in this line.

The total element content in the corn PGDH1 control and overexpression (Oex) lines was measured (Zm OexPGDH1-L2 and L3; see **Table 12**). For these experiments, only the two overexpressed lines showing the highest levels of protein in leaves and stems were chosen. The clearest changes were observed under elevated CO₂ (eCO₂) conditions. Under these elevated CO₂ growing conditions, the total nitrogen content in the leaves of wild type plants (WT) decreased while the carbon increased (**Table 12**). In stems, total nitrogen also decreased in wild type plants when grown under elevated CO₂ conditions. These changes resulted in a significant decrease in the nitrogen/carbon ratio in both organs. Furthermore, higher nitrogen (in leaves and stems) and sulphur (in leaves) content was found in Oex lines compared to wild type plants when plants were grown under elevated CO₂ conditions, leading to higher nitrogen/carbon (in leaves and stems) and sulphur/carbon (in leaves) ratio in Oex lines compared to controls (**Table 12**). Taken together, these results confirmed the role of PGDH1 protein in corn nitrogen homeostasis and show that this effect is more pronounced under elevated CO₂ conditions. Therefore, these results corroborate that using genetic engineering of the phosphorylation pathway of serine biosynthesis improves the nitrogen content of the crops.

**Table 12** shows the carbon, nitrogen and sulphur content (mg/g dry weight) and nitrogen/carbon and sulphur/carbon ratios in leaves and stems of corn lines overexpressing PGDH1 (Zm OexPGDH1-L2 and L3) grown under ambient CO₂ conditions or under elevated CO₂ conditions compared to wild type plants. The mean of the two overexpression lines (Zm OexPGDH1) is also shown. Values represent the mean ± SE of at least 6 groups of three plants per line. Different letters indicate significant differences between lines (P < 0.05) under the same growth conditions; significant differences between growth conditions according to Student's t-test are represented with * (P < 0.05). aCO2, ambient CO2 conditions; eCO2, elevated CO2 conditions; WT, wild type plants.

**Table 12**

| **Organ** | **Condition** | **Elements or proportion** | **Lines** | | | |
|---|---|---|---|---|---|---|
| | | | **WT** | **Zm OexPGDH1-L2** | **Zm OexPGDH1-L3** | **Zm OexPGDH1** |
| Leaves | aCO₂ | N | 37.78 ± 0.77 | 39.60 ± 0.78 | 37.93 ± 0.49 | 38.76 ± 0.47 |
| | | C | 410.79 ± 1.32 ab | 406.33 ± 1.74 b | 414.38 ± 1.56 a | 410.35 ± 1.34 ab |
| | | S | 2.41 ± 0.09 | 2.54 ± 0.09 | 2.50 ± 0.07 | 2.52 ± 0.06 |
| | | N/C | 9.21 ± 0.21 b | 9.76 ± 0.23 a | 9.16 ± 0.13 b | 9.46 ± 0.14 ab |
| | | S/C | 0.59 ± 0.02 | 0.63 ± 0.02 | 0.60 ± 0.02 | 0.61 ± 0.01 |
| | eCO₂ | N | 32.94 ± 0.61* c | 38.08 ± 0.69 a | 34.96 ± 0.73* bc | 36.57 ± 0.56* ab |
| | | C | 419.82 ± 1.39* a | 410.07 ± 1.56 c | 421.71 ± 0.99 a | 415.72 ± 1.36* b |
| | | S | 2.38 ± 0.10 b | 2.61 ± 0.10 ab | 2.70 ± 0.11 a | 2.66 ± 0.07 a |
| | | N/C | 7.85 ± 0.16* b | 9.30 ± 0.20 a | 8.30 ± 0.19* b | 8.81 ± 0.16* a |
| | | S/C | 0.57 ± 0.03 b | 0.64 ± 0.02 ab | 0.64 ± 0.03 a | 0.64 ± 0.02 a |
| Stems | aCO₂ | N | 35.06 ± 1.09 b | 38.27 ± 1.13 a | 36.46 ± 0.74 ab | 37.37 ± 0.69 ab |
| | | C | 363.87 ± 1.50 ab | 359.44 ± 1.61 b | 365.77 ± 1.50* a | 362.61 ± 1.21 ab |
| | | S | 1.80 ± 0.04 b | 1.93 ± 0.04 a | 1.84 ± 0.03 ab | 1.89 ± 0.03 ab |
| | | N/C | 9.65 ± 0.32 b | 10.67 ± 0.35 a | 9.98 ± 0.22 ab | 10.32 ± 0.21 ab |
| | | S/C | 0.50 ± 0.01 b | 0.54 ± 0.01 a | 0.50 ± 0.01 b | 0.52 ± 0.01 ab |
| | eCO₂ | N | 30.05 ± 0.94* c | 36.26 ± 0.80 a | 33.24 ± 1.00* b | 34.75 ± 0.68* ab |
| | | C | 363.41 ± 1.78 a | 349.50 ± 1.54* c | 362.27 ± 1.78 a | 355.89 ± 1.59* b |
| | | S | 1.95 ± 0.07 | 1.94 ± 0.06 | 2.00 ± 0.03* | 1.97 ± 0.04 |
| | | N/C | 8.29 ± 0.29* c | 10.39 ± 0.26 a | 9.20 ± 0.31* b | 9.79 ± 0.22 ab |
| | | S/C | 0.53 ± 0.02 | 0.56 ± 0.02 | 0.55 ± 0.01* | 0.55 ± 0.01* |

### Example 4. Characterisation of broccoli plants overexpressing the Arabidopsis thaliana PGDH1 gene

A construct expressing the cDNA of *Arabidopsis thaliana PGDH1* was prepared in broccoli plants and inserted into a plasmid. This plasmid was used to transform broccoli plants.

The contribution of overexpression of the *Arabidopsis thaliana* PGDH1 protein in broccoli plants, a species with C₃ metabolism, was studied. The protein content in transgenic broccoli plants was analysed in three overexpression lines of the *Arabidopsis thaliana PGDH1* gene.

All overexpression lines in T1 generation showed a higher protein content than the controls in the aerial part, between 8% and 17%. **Table 13** shows the analysis of all overexpression lines as a whole (Bo Oex PGDH1) with respect to wild type plant control (WT). In that case, the protein content of the aerial part of the overexpression lines increased significantly by 12% on average compared to the wild type plant control (WT) (**Table 13**). The material comes from plants grown in pots with perlite and Hoagland nutrient solution in the culture chamber for 45 days in the presence of 2000 ppms of CO₂. The values represent the mean expressed as a decimal to the wild type plant control (WT) ± SE (n ≥ 12 biological replicates). (*) represents significant differences to the wild type plant control (WT; T-test, P < 0.05).

**Table 13**

| **Lines** | **Relative content of the aerial part** |
|---|---|
| WT | 1.00 ± 0.02 |
| Bo Oex PGDH1 | 1.12 ± 0.02* |

### LIST OF BIBLIOGRAPHIC REFERENCES

Mizokami et al. (2019). Elevated CO2-induced changes in mesophyll conductance and anatomical traits in wild type and carbohydrate-metabolism mutants of Arabidopsis. J Exp Bot.;70(18):4807-4818. doi: 10.1093/jxb/erz208
Myers et al. (2019). Increasing CO2 threatens human nutrition. Nature;510(7503):139-42. doi: 10.1038/nature13179. Erratum in: Nature (2019);574(7778):E14.

## Claims

1. A genetically modified plant to overexpress a gene encoding a D-3-phosphoglycerate dehydrogenase (PGDH1) protein, wherein the amino acid sequence of said PGDH1 protein comprises the following amino acids:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348;
wherein said positions refer to the position in the amino acid sequence of the *Arabidopsis thaliana* PGDH1 protein identified by the sequence SEQ ID NO: 1; and wherein said plant does not belong to the *Arabidopsis thaliana s*pecies*.*

2. The plant according to claim 1, wherein the amino acid sequence of said PGDH1 protein further comprises the following amino acids:
- glycine at position 209;
- glycine at position 212;
- aspartate at position 256; and
- histidine at position 261;
wherein said positions refer to the position in the amino acid sequence of the *Arabidopsis thaliana* PGDH1 protein identified by the sequence SEQ ID NO: 1.

3. The plant according to claim 1 or 2, wherein, additionally, the amino acid sequence of said PGDH1 protein has an identity of at least 65% to the sequence SEQ ID NO: 1.

4. The plant according to any one of claims 1 to 3, wherein, additionally, the amino acid sequence of said PGDH1 protein has an identity of at least 75% to the sequence SEQ ID NO: 1.

5. The plant according to any one of claims 1 to 4, wherein, additionally, the amino acid sequence of said PGDH1 protein has an identity of at least 85% to the sequence SEQ ID NO: 1.

6. The plant according to any one of claims 1 to 5, wherein, additionally, the amino acid sequence of said PGDH1 protein has an identity of at least 95% to the sequence SEQ ID NO: 1.

7. The plant according to any one of claims 1 to 6, wherein the amino acid sequence of said PGDH1 protein further comprises a sequence selected from the group consisting of: SEQ ID NO: 3, SEQ ID NO: 4, SEQ ID NO: 5, SEQ ID NO: 6 and SEQ ID NO: 7.

8. The plant according to any one of claims 1 to 7, wherein the amino acid sequence of said PGDH1 protein further comprises a sequence selected from the group consisting of: SEQ ID NO: 16, SEQ ID NO: 17, SEQ ID NO: 18 and SEQ ID NO: 19.

9. The plant according to any one of claims 1 to 8, wherein the amino acid sequence of said PGDH1 protein is the sequence SEQ ID NO: 1.

10. The plant according to any one of claims 1 to 7, wherein the plant is from a species of agronomic interest.

11. The plant according to any one of claims 1 to 8, wherein the plant is from a species with C₃ metabolism.

12. The plant according to claim 9, wherein the plant is selected from tomato or broccoli.

13. The plant according to any one of claims 1 to 8, wherein the plant is from a species with C₄ metabolism.

14. The plant according to claim 11, wherein the plant is corn.

15. The plant according to any one of claims 1 to 12, wherein the gene encoding the PGDH1 protein is operably linked to a constitutive promoter.

16. The plant according to claim 13, wherein the constitutive promoter is selected from the constitutive promoter of the gene encoding corn ubiquitin and the constitutive promoter of cauliflower mosaic virus 35S.

17. A genetically modified plant cell to overexpress a gene encoding a D-3-phosphoglycerate dehydrogenase (PGDH1) protein, wherein said cell is genetically modified with an expression vector comprising a gene encoding a D-3-phosphoglycerate dehydrogenase (PGDH1) protein, wherein the amino acid sequence of said PGDH1 protein comprises the following amino acids:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348;
wherein said positions refer to the position in the amino acid sequence of the *Arabidopsis thaliana* PGDH1 protein identified by the sequence SEQ ID NO: 1; and wherein said cell is not from *Arabidopsis thaliana.*

18. The cell according to claim 17, wherein the amino acid sequence of said PGDH1 protein further comprises the following amino acids:
- glycine at position 209;
- glycine at position 212;
- aspartate at position 256; and
- histidine at position 261;
wherein said positions refer to the position in the amino acid sequence of the *Arabidopsis thaliana* PGDH1 protein identified by the sequence SEQ ID NO: 1.

19. The cell according to claim 17 or 18, wherein, additionally, the amino acid sequence of said PGDH1 protein has an identity of at least 65% to the sequence SEQ ID NO: 1.

20. The cell according to any one of claims 17 to 19, wherein, additionally, the amino acid sequence of said PGDH1 protein has an identity of at least 75% to the sequence SEQ ID NO: 1.

21. The cell according to any one of claims 17 to 20, wherein, additionally, the amino acid sequence of said PGDH1 protein has an identity of at least 85% with respect to the sequence SEQ ID NO: 1.

22. The cell according to any one of claims 17 to 21, wherein, additionally, the amino acid sequence of said PGDH1 protein has an identity of at least 95% with respect to the sequence SEQ ID NO: 1.

23. The cell according to any one of claims 17 to 22, wherein the amino acid sequence of said PGDH1 protein further comprises a sequence selected from the group consisting of: SEQ ID NO: 3, SEQ ID NO: 4, SEQ ID NO: 5, SEQ ID NO: 6 and SEQ ID NO: 7.

24. The cell according to any one of claims 17 to 23, wherein the amino acid sequence of said PGDH1 protein further comprises a sequence selected from the group consisting of: SEQ ID NO: 16, SEQ ID NO: 17, SEQ ID NO: 18 and SEQ ID NO: 19.

25. The cell according to any one of claims 17 to 24, wherein the amino acid sequence of said PGDH1 protein is the sequence SEQ ID NO: 1.

26. The cell according to any one of claims 17 to 25, wherein the cell is from a species of agronomic interest.

27. The cell according to any one of claims 17 to 26, wherein the cell is from a plant species with C₃ metabolism.

28. The cell according to claim 27, wherein the plant is selected from tomato or broccoli.

29. The cell according to any one of claims 17 to 26, wherein the cell is from a plant species with C₄ metabolism.

30. The cell according to claim 29, wherein the plant is corn.

31. A method for obtaining a genetically modified plant or a genetically modified plant cell to overexpress a gene encoding a D-3-phosphoglycerate dehydrogenase (PGDH1) protein, that comprises transforming the wild type plant or the corresponding plant cell with an expression vector comprising a gene encoding a D-3-phosphoglycerate dehydrogenase (PGDH1) protein, wherein the amino acid sequence of said PGDH1 protein comprises the following amino acids:
- leucine at position 65;
- leucine at position 78;
- arginine at position 108;
- serine at position 109;
- threonine at position 111;
- alanine at position 142;
- alanine at position 143;
- alanine at position 162;
- glutamic acid at position 163;
- arginine at position 291;
- glycine at position 292;
- leucine at position 300;
- aspartic acid at position 315;
- valine at position 316;
- phenylalanine at position 317;
- glutamic acid at position 320;
- proline at position 321;
- proline at position 338;
- histidine at position 339;
- glycine at position 341;
- serine at position 343;
- threonine at position 344;
- glutamic acid at position 346;
- alanine at position 347; and
- glutamine at position 348;
wherein said positions refer to the position in the amino acid sequence of the *Arabidopsis thaliana* PGDH1 protein identified by the sequence SEQ ID NO: 1; and wherein said plant or plant cell does not belong to the *Arabidopsis thaliana* species.

32. The method according to claim 31, wherein the amino acid sequence of said PGDH1 protein further comprises the following amino acids:
- glycine at position 209;
- glycine at position 212;
- aspartate at position 256; and
- histidine at position 261.
wherein said positions refer to the position in the amino acid sequence of the *Arabidopsis thaliana* PGDH1 protein identified by the sequence SEQ ID NO: 1.

33. The method according to claim 31 or 32, wherein, additionally, the amino acid sequence of said PGDH1 protein has an identity of at least 65% to the sequence SEQ ID NO: 1.

34. The method according to any one of claims 31 to 33, wherein, additionally, the amino acid sequence of said PGDH1 protein has an identity of at least 75% to the sequence SEQ ID NO: 1.

35. The method according to any one of claims 31 to 34, wherein, additionally, the amino acid sequence of said PGDH1 protein has an identity of at least 85% with respect to the sequence SEQ ID NO: 1.

36. The method according to any one of claims 31 to 35, wherein, additionally, the amino acid sequence of said PGDH1 protein has an identity of at least 95% with respect to the sequence SEQ ID NO: 1.

37. The method according to any one of claims 31 to 36, wherein the amino acid sequence of said PGDH1 protein further comprises a sequence selected from the group consisting of: SEQ ID NO: 3, SEQ ID NO: 4, SEQ ID NO: 5, SEQ ID NO: 6 and SEQ ID NO: 7.

38. The method according to any one of claims 31 to 37, wherein the amino acid sequence of said PGDH1 protein further comprises a sequence selected from the group consisting of: SEQ ID NO: 16, SEQ ID NO: 17, SEQ ID NO: 18 and SEQ ID NO: 19.

39. The method according to any one of claims 31 to 38, wherein the amino acid sequence of said PGDH1 protein is the sequence SEQ ID NO: 1.

40. The method according to any one of claims 31 to 39, wherein the plant or plant cell is from a species of agronomic interest.

41. The method according to any one of claims 31 to 40, wherein the plant or plant cell is from a species with C₃ metabolism.

42. The method according to claim 41, wherein the plant or the plant cell is selected from tomato or broccoli.

43. The method according to any one of claims 31 to 40, wherein the plant or plant cell is from a species with C₄ metabolism.

44. The method according to claim 43, wherein the plant or plant cell is from corn.

45. Use of the plant of any one of claims 1 to 16, or of the plant cell of any one of claims 17 to 30, for producing proteins, amino acids and/or starch.

46. Use according to claim 45, wherein the plant cell or plant is grown under conditions wherein the concentration of CO₂ is at least 800 ppm.
